# EUROPEAN PATENT SPECIFICATION

(11) **EP 2 680 744 B1**
(45) Date of publication and mention of the grant of the patent: **05.05.2021**
(21) Application number: 12755378.2
(22) Date of filing: 02.03.2012
(51) Int. Cl.: A61B 5/02, A61B 5/00, A61G 7/018, A61G 7/057, A61B 5/01, A61B 5/024, A61B 5/107, A61B 5/11

(54) **SENSING SYSTEM AND METHOD FOR PATIENT SUPPORTS**
SENSORSYSTEM UND VERFAHREN FÜR PATIENTENLIEGEN
SYSTÈME DE DÉTECTION ET PROCÉDÉ POUR SUPPORTS DE PATIENT

(30) Priority: 04.03.2011 US 201161449182 P
(43) Date of publication of application: 08.01.2014
(73) Proprietor: PatienTech LLC, Morning View, KY 41063 (US)
(72) Inventor: BALAKRISHNAN, Santoshkumar, Portage, Michigan 49024 (US); DAUGHERTY, Sean, Brookfield, Wisconsin 53045 (US); MIX, Joshua Elmer, Portage, Michigan 49002 (US); BHIMAVARAPU, Krishna Sandeep, Portage, Michigan 49024 (US); MROZ, Joseph E., Hastings, Michigan 49058 (US); KLINE, Edward C. III, Zeeland, MI 49464 (US); SHEA, Xianyu, Vicksburg, MI 49097 (US); DERENNE, Richard A., Portage, Michigan 49002 (US)
(74) Representative: FRKelly
(86) International application number: PCT/US2012/027402
(87) International publication number: WO 2012/122002

(56) References cited:
- DE-A1-102005 048 496
- FR-A1- 2 728 775
- US-A- 5 571 973
- US-A- 5 873 137
- US-A- 5 993 400
- US-A1- 2004 237 202
- US-A1- 2005 128 184
- US-A1- 2005 165 284
- US-A1- 2005 268 401
- US-A1- 2006 277 683
- US-A1- 2007 179 360
- US-A1- 2008 169 931
- US-A1- 2008 235 872
- US-A1- 2008 303 899
- US-A1- 2009 183 312
- US-A1- 2010 018 327
- US-A1- 2010 094 175
- US-A1- 2011 047 706
- US-B1- 6 721 980

## Description

This application claims priority to U.S. provisional patent application Ser. No. 61/449,182 filed March 4, 2011, by applicant Richard Derenne and entitled Sensing System for Patient Supports.

### BACKGROUND OF THE INVENTION

The present invention relates to medical devices, and more particularly to a sensing system used in conjunction with patient supports, such as beds, stretchers, cots, gurneys, operating tables, recliners, chairs, and the like.

US 5,571,973 discloses a sensor array for measuring shear forces.

### SUMMARY OF THE INVENTION

A sensing system according to the invention is defined in claim 1. A method according to the invention is defined in claim 13. Furthermore, the following is disclosed:
According to its various embodiments, the present invention provides an improved sensing system that provides information about a patient and/or a patient support apparatus, such as a bed, stretcher, cot, operating table, chair, recliner, or other type of patient support. In one embodiment, the sensing system may be applied to existing patient supports to turn previously existing beds into smart beds, e.g. beds that provide information about components of the bed and/or aspects of the patient to a remote location. In other embodiments, the sensing system provides an improved tool for assisting in the prevention and/or management of bed sores. In still other embodiments, the sensing system provides additional data about the patient and/or the patient support that is useful to a clinician or caregiver. In at least one embodiment, the sensing system includes a flexible sheet that houses a sensor array and that is adapted to be releasably secured to a patient support surface, such as a mattress, an array of air bladders, or other type of cushioned support on which a patient may rest. In another embodiment, the flexible sheet may be incorporated into a patient support surface, such as a mattress, or the like. Various additional embodiments and features are described below.

According to one embodiment, a sensing system is provided that includes a sensor array, a controller, a pedestal, and a user interface. The sensor array is adapted to be placed over a patient support surface positioned on the patient support. The sensor array detects force exerted by a patient onto the sensor array. The controller communicates with the sensor array and processes outputs from the sensor array to determine a patient-related parameter. The pedestal is supportable on an IV hole defined in the patient support. The user interface is supported on the pedestal and is in communication with the controller. The user interface allows a caregiver to control an alert related to the patient-related parameter.

According to another embodiment, a sensing system for a patient support is provided that includes a sensor array, a controller, a display screen, and a user interface. The sensor array is integrated into a flexible mat that is adapted to be placed over a patient support surface positioned on the patient support. The sensor array includes a plurality of sensors that are each adapted to detect force exerted by a patient onto a respective sensor in the sensor array. The controller communicates with the sensor array and processes outputs from the sensor array to determine a patient-related parameter. The display screen is supported on the patient support. The user interface is supported on the patient support and associated with the display screen. The user interface communicates with the controller and allows a caregiver to select which of the plurality of sensors in the sensor array are used in at least one calculation performed by the controller.

According to another embodiment, a system is provided that includes a first sensor array, a second sensor array, a controller, and a display screen. The first sensor array is incorporated into a first flexible mat, and the second sensor array is incorporated into a second flexible mat. The controller is adapted to receive outputs from both the first and second sensor arrays and integrate them into a combined pressure distribution profile of at least a portion of a patient's body. The controller forwards the combined pressure distribution profile to a display for viewing by a caregiver.

According to another embodiment, a method is provided for upgrading a patient support that does not communicate data to a remote location to an upgraded patient support that does communicate data to a remote location. The method includes providing a flexible mat having a sensor array integrated therein. The sensor array includes a plurality of sensors that are each adapted to detect force exerted by a patient onto a respective sensor in the sensor array. The method further includes placing the mat over a patient support surface positioned on the patient support; providing a controller separate from the patient support to process outputs from the sensor array to determine a pressure distribution profile of the patient; displaying the pressure distribution profile of the patient on a display screen supported on the patient support; and communicating the pressure distribution profile of the patient off the bed to a remote location.

According to other aspects, the method may further include using the sensor array and the controller to detect when a patient has exited the patient support; and communicating an alert signal from the patient support to the remote location indicating that the patient has exited the patient support. The method may also include providing an angle sensor within the mat adapted to determine an angle of at least one pivotable section of the patient support; and communicating the angle of the pivotable section to the remote location.

In still other embodiments, the controller of the system may be adapted to determine from the sensor array whether a patient has turned or not, or whether a patient has sat up on the patient support, or if an undesirable condition exists with respect to shear pressure exerted between the patient and the patient support surface.

The sensor array may include a tilt sensor and the controller may be adapted to determine an angular orientation of a pivotable portion of the patient support surface. The tilt sensor may be an accelerometer, or other sensor that is suitable for determining an angular orientation. The angular orientation may be with respect to gravity, or with respect to another reference.

The sensing system may be adapted to sense an undesirable condition relating to shear pressure by detecting movement along the sensor array of an area of pressure exerted by the patient against the sensor array that exceeds a threshold size and magnitude. The sensing system may also be adapted to detect multiple types of different undesirable shear pressure conditions. For example, in addition to the movement along the sensor array of an area of pressure exceeding a threshold size and magnitude, the sensing system may be adapted to detect an undesirable shear condition that includes a threshold amount of patient weight moving back and forth along the sensor array for a certain amount of time.

According to other embodiments, the user interface may include a display screen supported on the pedestal. An alert light may also be supported on the pedestal or incorporated into the bed or positioned elsewhere. If supported on the pedestal, the alert lamp may be spaced apart from the display screen. The alert lamp, wherever positioned, may be sized and positioned such that it is visible from a hospital hallway when the patient support is positioned within a room such that a caregiver does not need to come close to the patient support to determine if an undesirable condition exists with respect to the sensing system or not. That is, the alert lamp may stand apart-in terms of size and/or position and/or in other ways-from the regular lights that appear on one or more of the patient support's control panels or a control panel associated with the user interface of the sensing system. Such regular lights, whether showing up on a display screen, or places adjacent to a display screen, typically are intended to be viewed by a patient or caregiver who is positioned immediately next to the lights (e.g. by a patient on the patient support or a caregiver standing next to the support and interacting with the control panel). The alert lamp(s) associated with one embodiment of the present invention are, in contrast, intended to allow a caregiver to simply and immediately view from a hallway, or other significant distance, whether an undesirable condition exists or not, without requiring the caregiver to walk all the way up to the patient support.

In still other embodiments, the controller of the system may determine from the sensor array if a patient has bottomed out on a surface positioned underneath the patient. The controller may also determine if a patient has exited the patient support.

The sensor array may be integrated into a flexible mat adapted to be fitted over a surface positioned on the patient support, or a portion of a surface on the patient support, or one or more pillows or positioners positioned on the patient support. In still other embodiments, multiple flexible mats with sensor arrays may be provided and positioned over multiple of these structures.

The sensor array may be made up of a plurality of piezoresistive cells that each have an electrical resistance that changes in response to a force exerted thereon.

A mounting bracket may be included that is configured to be inserted into the IV hole on the patient support wherein the bracket supports the pedestal and includes a hole for supporting an IV pole. In this manner, both the pedestal and an IV pole may share a single, common IV hole on a patient support. The pedestal may include a boom that is releasably attachable to the mounting bracket. The pedestal may further be adapted to be pivotable about both a horizontal and a vertical axis.

The system may include an identification card reader adapted to read identification cards carried by authorized caregivers. The use of such identification cards may limit access to the system such that only authorized caregivers can change a setting of the system via the user interface. The use of such identification cares may also enable the recording of an action taken by an authorized caregiver whose identification card is read by the identification card reader. Such actions might include turning a patient or other actions.

The system may include, in still other embodiments, a wireless transceiver that communicates wirelessly with a communication network and that forwards an alert signal to the communication network. The communication network may be an existing hospital computer network, such as an Ethernet or other computer network, that ties together electronic medical records and/or other information.

A location device may be included with the system that is supported on the patient support and that communicates wirelessly with a nearby transmitter having an identity correlated to a specific location within a health care facility. The location device will thereby allow a determination of the location of the system within a particular medical facility to be made. Information transmitted from the patient support to remote locations can thereby be correlated to specific locations within the medical facility.

The user interface may include a display screen on which a graphical depiction of a location and magnitude of forces exerted by a patient against the sensor array can be depicted. A memory may further be included for storing the outputs from the sensor array over time. The user interface may interact with the memory and display screen to display a plurality of past graphical depictions of outputs from the sensor array. The display of the past graphical depictions may be done in a movie-like manner in which the graphical depictions either continuously change at a rate that matches the actual past pressure changes, or at an accelerated rate in which the graphical depictions continuously change at a rate greater than the actual rate of change of the past pressure changes. A graphical timeline may be included on the display screen that includes indications of past events detected by the sensor array.

The controller may be configured to issue an alert when any one or more of the following conditions occurs: a patient may need boosting on the patient support, a patient may need to be turned, a patient may be experiencing excessive shear stress, a patient may be experiencing excessive compressive stress, a patient may have changed from a supine to a sitting position, a patient may have exited the patient support, and an angle of a head section of said patient support may have decreased below a threshold. The user interface may allow a caregiver to select any one or more of these conditions that, either alone or in combination with others of these conditions, will cause the alert.

The controller may be configured to determine a patient's height when the patient is resting on the sensor array. The controller may also be configured to determine a patient's body mass index when the patient is resting on the sensor array. Still further, the controller may be configured to determine from the sensor array whether a non-patient object is positioned on the sensor array. The controller may also determine a patient's orientation on the sensor array based upon outputs from the sensor array, or the controller may determine from the sensor array if an entrapment hazard exists for a patient positioned on support surface.

The patient-related parameter detected by the sensor array may include any one or more of the following: a patient's heart rate, a patient's breathing rate, a patient's temperature, a patient's height, a patient body mass index, a patient's regional mass index, a patient's weight, a patient's quality of sleep, and a patient's level of agitation.

Additional sensors may be included with the sensor array or within the flexible mat. Such additional sensors may include one or more of the following: a temperature sensor, a moisture sensor, an accelerometer or other angle sensor, a pulse oximeter, and a side rail position sensor. If included, the temperature sensor may be used to adjust force values detected by the sensor array in order to compensate for temperature-dependent errors in the value output by the sensor array.

In some embodiments, the sensor array may include over a thousand sensor cells that are each adapted to detect a force exerted by a patient onto a corresponding cell area of the sensor array. The sensor array may be integrated into a mattress cover that covers a mattress, or other type of surface, positioned on the patient support. Alternatively, the sensor array may be integrated into the mattress itself, in some embodiments.

The sensor array, controller, and user interface may receive electrical power from an electrical transformer that also supplies electrical power to a surface on the patient support. The user interface may include a touch screen display that allows a caregiver to control the system by pressing the touch screen.

The sensing system may communicate with a scale system on the patient support wherein the scale system measures a total weight of items positioned on at least a portion of the patient support. The user interface may allow a caregiver to select a portion of the sensor array such that the user interface thereafter provides an indication of the total weight sensed by the sensor array in the selected portion. The sensor array may also include a section dedicated to determining a weight of non-patient objects placed on the section.

The system controller may communicate with a surface controller that controls a surface positioned on the patient support and underneath the sensor array. The system controller may communicate force and location information from said the array to the surface controller.

The system may also be used to determine undesired shear stresses. The system may issue an alert if the sensor array detects an undesired degree of stress exerted on a patient supported in the patient support. The user interface may enable a caregiver to exclude one or more areas of the sensor array when the controller determines if an alert condition is present. Such exclusion may allow the caregiver to avoid pressure alerts that might otherwise be generated as a result of pressure exerted on a patient's cast, or caused by other conditions that are not likely to lead to pressure ulcer development.

The system may further be configured to map pressure readings from the sensor array to specific patient body portions. The controller may use the mapping of the pressure readings to the specific patient body portions when determining the patient-related parameter. The controller may process signals from the sensor array corresponding to the location of a specific body portion differently from signals from the sensor array corresponding to a different location. For example, the controller may determine a location of a patient's torso on the sensor array and use the knowledge of the location of the patient's torso when determining a heart rate or a respiration rate of the patient.

The system may include a pivotable display that displays an image having a desired orientation and the controller may be adapted to automatically change an orientation of the image in response to pivoting of the display so that a desired orientation of the image is maintained before and after the pivoting of the display. The image displayed may include a graphical representation of a pressure distribution of the pressure exerted on the patient. The image may also include an outline of the patient in conjunction with the graphical image.

In some embodiments, notes may be entered by a caregiver into the system through the user interface whereby the notes are recorded in a memory for later viewing.

A communication port may be included that allows the controller to communicate data gathered from the sensor array to a display positioned off of the patient support. The patient support may be one of a bed, a cot, a stretcher, an operating room table, a recliner, a chair, or the like. A portion of the sensor array may define a pressure sensitive keypad that may be pressed by a caregiver to control a feature of the sensing system. The pressure sensitive keypad may include indicia that are projected onto a surface over the sensor array by a light projecting device.

The controller may be divided into multiple components and may include a first portion physically attached to the sensor array and a second portion physically attached to a display portion of the user interface. The display may include a zoom feature that allows a caregiver to zoom into images displayed thereon-such as pressure distribution images-thereby enabling the caregiver to see the pressure distribution images with a larger size.

In other embodiments, the system may include multiple sensor arrays. The first sensor array and second sensor array may be placed in positions that are separate from each other such that the second sensor array contacts a portion of a patient's body not in contact with the first sensor array. The multiple sensor arrays may both generate data that is correlated to each other and displayed on a display supported on the pedestal. The display of data may be done in such a manner such that a common graphical image is shown on the display that includes data from both of the multiple sensor arrays, or, in some embodiments, from more than two sensor arrays. The common graphical image may be a pressure distribution image, or some other type of common graphical image.

The sensor array may be integrated into a flexible mat. The sensor array may be configured such that it includes a substantially continuous array of sensor cells that extend from a first side of the patient support to a second side of the patient support, and from a head end of the patient support to a foot end of the patient support. Thus, the sensor array can detect pressure applied to all, or substantially all, of the top of the patient support surface.

The controller may be configured to use the sensor array to determine a weight of a patient positioned on the sensor array and to communicate the weight to a scale system on the patient support whereby the patient weight may be used to determine what portion, if any, of the weight detected by the scale system is non-patient weight. The patient weight measured using the sensor array may also be used by the scale system to perform an automatic zeroing function.

One or more additional footboard sensors may be included that detect force exerted against any portion of a patient's body that comes into contact with the footboard. When so included, the footboard pressure sensors may communicate with the controller such that data regarding the footboard pressure is displayed on the display associated with the user interface.

One or more pillow pressure sensors may also be included that detect forces exerted by the patient onto the pillow. When so included, the pillow pressure sensor communicates with the controller and the system displays information gathered from the pillow pressure sensor the display associated with the user interface. When the pillow pressure sensor is included, the controller may also integrate first data from the sensor array and second data from the pillow pressure sensor into a common graphical image displayed on the display. The sensor array may include a sensing region large enough to detect the pillow positioned thereon, and the controller may use the detected position of the pillow on the sensor array to align a pressure distribution detected by the sensor array with a pressure distribution detected by the pillow pressure sensor. The pillow pressure sensor may be a flexible mat adapted to envelope the pillow. The pillow pressure sensors may also include one or more reference objects that are detectable by a sensor in the sensor array such that the controller may use the reference object to determine an alignment of the pillow pressure sensor with the sensor array.

In other embodiments, a footboard sensor may be included that detects whether a footboard has been removed from the patient support and indicates to a caregiver if the footboard is removed from the patient support. Alternatively, or in addition, a headboard sensor that detects whether a headboard has been removed from the patient support and that provides an indication to the caregiver if the headboard is removed from the patient support may be provided.

The system may include an angle sensor adapted to detect an actual turn angle of a patient undergoing a caregiver-assisted turn. Such an angle sensor may include one or more accelerometers, or other types of angle sensors. The system may also include a plurality of sensors adapted to determine whether a plurality of side rails on the patient support are in a raised position or not, and to communicate such information to a caregiver. The system may further include a mobile camera adapted to be releasably coupled to the pedestal.

The user interface of the system may enable a caregiver to enter a Braden scale score and the controller, in response thereto, may automatically choose at least one alert criteria based said Braden scale. The user interface may further be adapted to allow a caregiver to override the at least one automatically chosen alert criteria. The alert criteria may include both a time component and a pressure magnitude component.

The system may include a light projector supported on the patient support that projects color coded light onto a patient wherein the color coding indicates degrees of pressure experienced by the patient on the mat.

The user interface may enable a caregiver to select an area on the sensor array that, in the absence of any patient force detected on the selected area, will cause the user interface to indicate an alert condition.

The sensor array may be incorporated into a flexible mat that includes both a temperature sensor and a moisture sensor. When the temperature and moisture sensors are included, the controller may communicate with a controllable surface having inflatable chambers positioned underneath the sensor array in such a manner so as to cause at least one of the inflatable chambers to change its inflation pressure in response to temperature and moisture values output from the temperature and moisture sensors.

The outputs from the sensors array and/or any other sensors included in the system may be communicated wirelessly to a physical device that contains the user interface, or that contains the display. Such wireless communication avoids the need for supplying and arranging one or more wired connections between the sensor(s) and the user interface and/or display.

A printer may be included that allows a caregiver to print out a paper image representing data gathered from the sensor array.

When the sensor array is incorporated into a flexible mat, the flexible mat may further include a fluid inflatable chamber in fluid communication with a plurality of air holes defined in the bottom side of the mat wherein the inflatable chamber and air holes are adapted to enable a caregiver to selectively create an air cushion underneath the mat for facilitating the transfer of a patient from one patient support surface to another.

The user interface may allow a caregiver to select an area on the sensor array that, in the presence of a threshold amount of patient movement detected on the selected area, will cause the user interface to provide an indication discernible to the caregiver. The area selectable by the caregiver may be one or more of fixed areas, or it may include non-fixed areas that are definable by the caregiver to enable customization of the area's boundaries.

The system may be useful during physical therapy procedures by determining one or more parameters that are useful for a caregiver during the physical therapy procedure, and by further forwarding the parameters, or information related to the parameters, to a display associated with the user interface.

The system may keep track of patient pressure readings and movement over extended periods of time so as to further assist the caregiver. For example, the controller may store sensor array data in a memory, analyze the data to determine at least one patient tendency, and provide a recommendation to the caregiver about an action the caregiver can take to provide better care for the patient in light of the patient tendency. The time over which such analyzed data may be gathered may be hours, days, or other amounts of time. The patient tendency includes any one of the following, or still other tendencies: sleeping on a particular side of the patient support, using more than one pillow, or exiting the patient support from a particular location.

A camera may be included with the system to take pictures of patient's position in order to assist the controller in correlating the patient's body to the pressure profile determined from the sensor array. The pictures may be used to map the outputs from the sensor array to specific areas of the patients' body. Alternatively, the mapping may be carried out in other manners that do not involve a camera.

The user interface may allow a caregiver to input a picture of a patient's wound or bed sore into memory accessible to the controller. The inputted picture may then be used by the system in such a manner that the display displays both the picture of the wound or bed sore and a pressure distribution profile determined by the controller from the outputs of the sensor array. This would enable the caregiver to see the relationship between the wound or bed sore and the pressure experienced by the patient so as to assist the caregiver in avoiding excessive pressure being applied to the wound or bed sore.

The controller may analyze the outputs from the sensor array over time and provide a recommendation to a caregiver of a type of surface to be used on the patient support. The recommendation may be based upon information stored in a memory accessible to the controller that indicates which types of surfaces are available at the particular medical facility in which the patient support is located. The controller may also analyze the outputs from the sensor array over time and provide a recommendation to a caregiver of a type of patient support to be used with the patient. The recommendation may be based upon information stored in a memory accessible to the controller that indicates which types of patient supports are available at the particular medical facility in which the patient support is located.

The controller may communicate pressure data to a surface controller that controls a surface positioned on the patient support in order to enable the surface controller to target a surface treatment procedure to a specific area of a patient's body.

The controller may also be configured to use the outputs of the sensor array to determine a patient's location on the sensor array, and to use the patient's location to define a plurality of zones on the sensor array, wherein the user interface allows a caregiver to select at least one of the zones to base an alert condition on. The controller may also dynamically adjust the boundaries of the zones if the patient changes location on the patient support surface.

The display may be a pivoting display mounted on the footboard of the patient support, or elsewhere, that automatically changes the images shown thereon based upon the current pivot position of the display. For example, the display may automatically change its content depending upon whether or not the display is facing toward the patient or away from the patient. Alternatively, the display may automatically chance the orientation of information displayed thereon in response to pivoting of the display. Or the display may make both content and orientation changes in response to pivoting.

In still other embodiments, the sensor array may be incorporated into a flexible sheet that includes an adhesive, or other fastening structure, for attaching the sheet to a patient, or a portion of a patient's body. The sheet may then be used to monitor forces experienced by that particular portion of the patient's body.

It will be understood that all of the various features recited herein in the "Summary of the Invention" section may be incorporated in any possible combinations or permutations.

### BRIEF DESCRIPTION OF THE DRAWINGS

FIG. 1 is a block diagram of a sensing system according to a first embodiment;
FIG. 2 is a block diagram of a sensing system according another embodiment;
FIG. 3 is a block diagram of a sensing system according to still another embodiment;
FIG. 4 is a diagram of a sensing system according to still another embodiment that includes an illustrative bed and more detail than the drawings of FIGS. 1-3;
FIG. 5 is a perspective view of a patient support that may be used with a sensing system showing a tablet of the patient support mounted at a head end of the patient support;
FIG. 6 is a perspective view of a patient support that may be used with a sensing system showing a tablet of the patient support mounted at a foot end of the patient support;
FIG. 7 is a plan view diagram of one example of a sensor array that may be used in any of the embodiments disclosed herein;
FIG. 8 is a system diagram of an illustrative hospital network with which the sensing system may be in communication;
FIG. 9 is an illustrative screen shot generated by the sensing system that may be displayed and which illustrates various features of one embodiment of the sensing system;
FIG. 10 is another illustrative screen shot generated by the sensing system that may be displayed in response to a user selecting the "timeline" control of FIG. 9;
FIG. 11 is an illustrative screen shot generated by the sensing system that may be displayed in response to a user selecting an "events" control and a "snap shot" control in FIG. 10;
FIG. 12 is another illustrative screen shot generated by the sensing system that may be displayed in response to a user selecting the "status" control of FIG. 9;
FIG. 13 is an illustrative screen shot that may be generated by the sensing system;
FIG. 14 is another illustrative screen shot that may be generated by the sensing system that has different options than the screen shot of FIG. 13;
FIG. 15 is another illustrative screen shot that may be generated by the sensing system;
FIGS. 16-18 are additional illustrative screen shots that may be generated by the sensing system;
FIG. 19 is an illustrative screen shot that may be generated illustrating various controls, settings, and/or functions that may be configured by a user of the system;
FIG. 20 is an illustrative screen shot that may be generated when a new patient is going to be monitored by the sensing system;
FIG. 21 is a perspective view of an illustrative tablet of the sensing system, showing one manner in which an alert light may be incorporated thereinto;
FIG. 21A is a partial, cross-section of an edge of the tablet of FIG. 21;
FIG. 21B is a partial, cross-section of an edge of the tablet of FIG. 21 showing a cover moved to a different position than in FIG. 21A;
FIG. 22 is a perspective view of a tablet showing an alternative embodiment of an alert light;
FIG. 22A is a partial, cross-section of an edge of the tablet of FIG. 22;
FIG. 23 is a perspective view of a tablet showing another embodiment of an alert light;
FIG. 23A is a partial, cross-section of an edge of the tablet of FIG. 23;
FIG. 24 is a perspective view of a tablet showing another embodiment of an alert light;
FIG. 24A is a partial, cross-section of an edge of the tablet of FIG. 24;
FIG. 25 is a perspective view of a tablet showing another embodiment of an alert light;
FIG. 26 is a perspective view of a tablet showing another embodiment of an alert light;
FIG. 27 is a perspective view of a tablet showing yet another embodiment of an alert light;
FIG. 27A is a partial, cross-section of an edge of the tablet of FIG. 27;
FIG. 28 is a perspective view of a patient support showing a tablet and pedestal of the sensing system mounted thereto;
FIG. 29 is a perspective view of an embodiment of a tablet with a display screen having an illustrative screen shot displayed thereon;
FIG. 30 is a perspective, exploded view of one manner of constructing a tablet of the sensing system;
FIG. 31 is a series of side, elevation views of a patient support in different orientations to which a pedestal and tablet of the sensing system is mounted, illustrating the various mounting positions and orientations of the pedestal and tablet;
FIG. 32 is a perspective, exploded view of one embodiment of a pedestal;
FIG. 33 is a perspective, exploded view of a second embodiment of a pedestal;
FIG. 34 is a perspective view of a mounting bracket for the pedestal;
FIG. 35 is a plan view of a conventional IV pole support hole defined in a patient support;
FIG. 36 is a diagram of a switch mechanism that cooperates with the support bracket;
FIG. 37 is a perspective view of the mounting bracket inserted into an IV pole support hole of a patient support;
FIG. 38 is a plan view of the mounting bracket of FIG. 37 inserted into the IV pole support hole;
FIG. 39 is a schematic view of an illustrative manner in which cables may be configured for supplying data and power to the sensing system;
FIG. 40 is diagram illustrating how a male sized in the 95^{th} percentile and a female size in the 5^{th} percentile fit on a mattress that is eighty-four inches long;
FIG. 41 is a diagram illustrating a number of zones that may be defined for a sensor array, including their relationship to the male and female outlines of FIG. 40; and
FIG. 42 is a diagram illustrating the female outline of FIG. 40 slide downward on the sensor array.

### DETAILED DESCRIPTION OF THE EMBODIMENTS

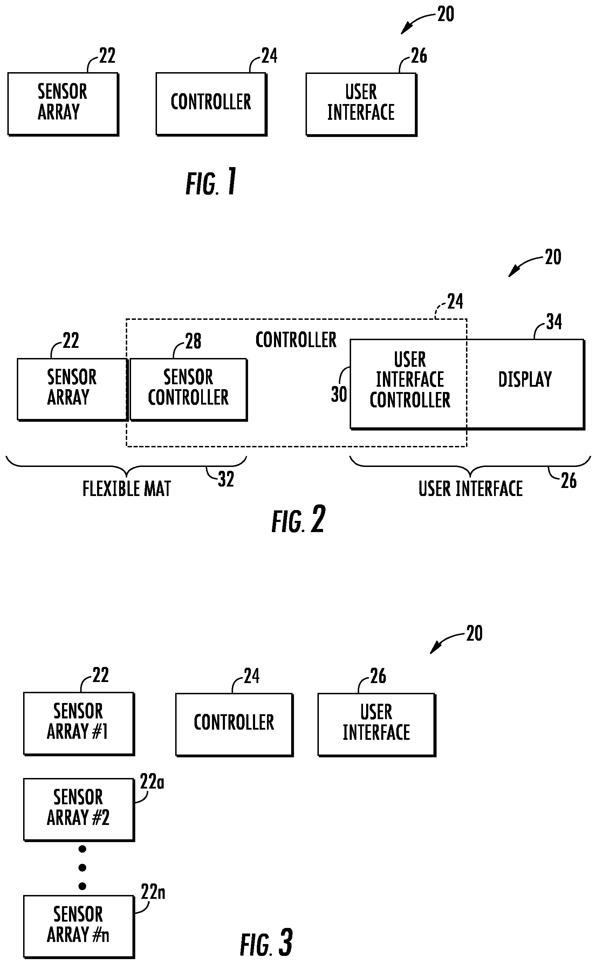

A sensing system 20 according to a first embodiment is depicted in block diagram form in FIG. 1. Sensing system 20 is used to sense one or more parameters that relate to a patient who may be positioned on a patient support apparatus, such as a bed, a stretcher, a cot, a recliner, an operating room table, or other patient support apparatus. In many embodiments, sensing system 20 detects pressure exerted by the patient against a surface. The detection of pressure may be used for multiple purposes including, but not limited to, the treatment and/or prevention of pressure ulcers on a patient. Other uses are also possible, as will be described in more detail below.

In the embodiment illustrated in FIG. 1, sensing system 20 includes a sensor array 22, a controller 24, and a user interface 26. The sensor array 22 may be incorporated into a flexible mat that is configured to be positioned on top of a mattress, or other type of patient support surface. A sheet, or other material, may be placed over the mat and when a patient sits, lies, or otherwise rests on top of all or a portion of the mat, the pressure exerted by the patient onto the mat will be detected by the sensor array 22 incorporated therein. In other embodiments, the mat may be positioned on top of other structures, such as a pillow, a patient positioner, the floor, a piece of furniture separate from the hospital bed, or some other object. The details of various illustrative constructions of the sensor array will be discussed in more detail below.

The controller 24 may include one or more microprocessors, microcontrollers, systems-on-a-chip (SoC), field-programmable gate arrays, application specific integrated circuits (ASICs), any types of programmable logic devices (PLDs), discrete logic circuits, or any other electronic structure or combinations of electronic structures capable of carrying out the algorithms discussed herein, as would be known to one of ordinary skill in the art. Such algorithms may be carried out in software, firmware, or dedicated hardware, or any combination of these. Controller 24 may be comprised of multiple components that are located at different physical locations, including one or more components positioned physically inside a first device, one or more additional components positioned inside a second device, and possibly additional components positioned inside other devices. For example, in the embodiment illustrated in FIG. 2, controller 24 includes a sensor controller component and a user interface controller component. The two components may be located in different devices or housings that are separated from each other, but in communication with each other via either wireless communication or one or more communication wires. In other embodiments, controller 24 may be entirely located within a single device or housing. Other details about the structure, functions, and construction of controller 24 will be provided below.

User interface 26 enables a user, such as a caregiver, to control one or more aspects of sensing system 20. User interface 26 may take on multiple different forms. In one embodiment, user interface 26 may include a keypad to enable a user to manipulate sensing system 20. In another embodiment, user interface 26 may include a touch screen that allows a caregiver to both visually see information presented on the touch screen display and to input control instructions and/or data by physically pressing the appropriate locations on the touch screen. In other embodiments, a non-touch screen display may be provided that displays information about sensing system 20 to the caregiver and/or the patient. Such a display screen may include any conventional display technology, such as a liquid crystal display (LCD), a plasma display, a cathode ray tube, or any other suitable display technology.

FIG. 2 illustrates another embodiment of sensing system in which controller 24 is divided into two physically separate components: a sensor controller 28 and a user interface controller 30. Further, in the embodiment of FIG. 2, sensor array 22 is incorporated into a flexible mat 32 that either incorporates, or is attached to, sensor controller 28. Sensor controller 28 processes the outputs from sensor array 22 and forwards the processed results to user interface controller 26. User interface controller 30, in the embodiment illustrated in FIG. 2, is incorporated into the same physical structure as a display 34. Together, display 34 and user interface controller 24 form all or a portion of user interface 26. It will be understood by those skilled in the art, however, that user interface controller 30 could be physically separate from display 34. It will also be understood that user interface 26 could include additional structures not illustrated in FIG. 2, such as, for example, a keypad, one or more switches, buttons, or other structures that allow a caregiver to control one or more aspects of sensing system 20. Still further, it will be understood that, although FIG. 2 illustrates sensor controller 28 physically attached to, or incorporated within, flexible mat 32, sensor controller 28 could be physically separate from flexible mat 32-either incorporated wholly into user interface 26, or wholly separate from user interface 26 in a stand-alone structure, or divided between multiple structures.

As with controller 24, each controller component 28 and 30 may include one or more microprocessors, systems-on-a-chip (SoC), field-programmable gate arrays, discrete logic circuits, or any other electronic structures or combinations of electronic structures capable of carrying out the algorithms discussed herein, as would be known to one of ordinary skill in the art. Such algorithms may be carried out in software, firmware, or dedicated hardware, or any combination of these.

FIG. 3 illustrates a block diagram of yet another embodiment of sensing system 20. Sensing system 20 of FIG. 3 differs from the embodiments shown in FIGS. 1 and 2 in that it includes multiple sensor arrays 22 that communicate with a single controller 24. The total number of sensor arrays 22 that communicate with controller 24 in this embodiment may vary from two sensor arrays up to an arbitrary number n. The multiple sensor arrays 22 may all be positioned on a single patient support, such as a bed, cot, stretcher, etc.; or one or more of them may be positioned on other patient support structures, such as a recliner or an operating table, or on even other structures, such as the floor. If more than one array 22 is positioned on a single support structure, the multiple sensors may be adhered to a common structure, such as a mattress or other patient support surface, or they may be adhered to different structures-such as one or more adhered to pillows, patient positioners, footboards, headboards, side rails, and/or the patient support surface. As another example, if multiple arrays 22 are used on a recliner, one array may be positioned on a back rest portion of the recliner, another may be positioned on a seat rest portion of the recliner, and yet another array 22 may be positioned on a foot rest portion of the recliner.

While FIG. 3 illustrates the multiple sensors arrays 22 communicating with a single, common controller 24, it will be understood that controller 22 in FIG. 3 may be divided into multiple individual components, just as in the other embodiments. Thus, for example, each sensor array 22 in the embodiment of FIG. 3 may include a sensor controller component 28 that talks to another controller component wherein the two or more controller components together make up controller 24. Multiple user interfaces 26 could also be used, although a single user interface that allows control over multiple sensor arrays 26 may be more desirable in some embodiments.

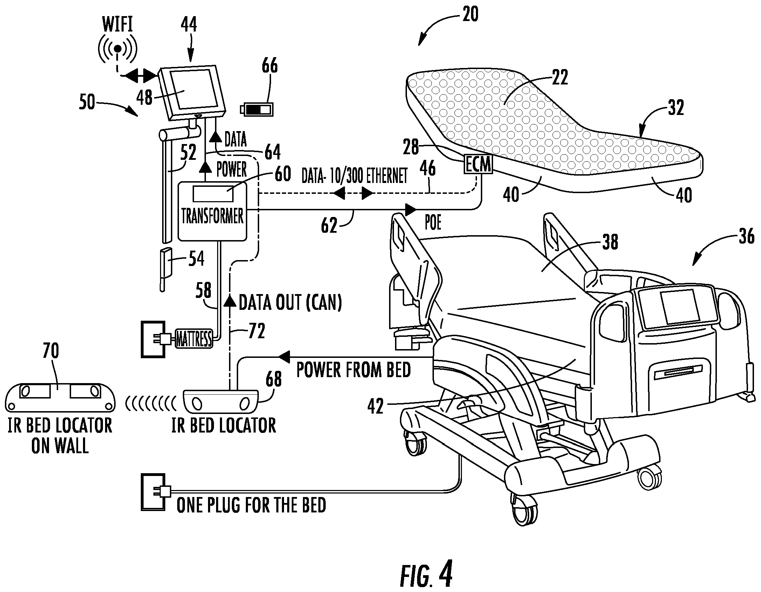

FIG. 4 provides a more detailed overview of another embodiment of sensing system 20 and a patient support 36 with which sensing system 20 may be used. In the embodiment of FIG. 4, sensor array 22 is incorporated into a flexible mat 32 that is shaped to fit over a mattress 38, or other support surface, supported on patient support 36. Flexible mat 32 may be integrated into a mattress cover that fits over mattress 38, or it may be a separate structure that is positioned on top of the mattress cover. In the embodiment shown in FIG. 4, flexible sheet 32 is physically shaped in a manner similar to a conventional fitted sheet. That is, it includes side sections 40 that fit over all or a portion of the sides 42 of mattress 38. An elastic band, draw string, Velcro® or other type of structure may be used to secure flexible mat 32 to mattress 38. Such a structure may also assist in maintaining a top surface 44 of flexible mat 32 in a generally smooth state such that no portions of flexible mat 32 are folded over on each other or bunched together. This helps avoid sensing errors that may otherwise occur if mat 32 is folded onto itself or bunched together.

In the embodiment of FIG. 4, controller 24 is split amongst a sensor controller 28 and a user interface controller 30 incorporated inside of a tablet 44. Sensor controller 28 is labeled as EDM in FIG. 4, which stands for electronic control module. While sensor controller 28 is shown attached to, or incorporated inside of, a side section 40 of flexible mat 32, it will be understood that sensor controller 28 may be positioned elsewhere. Further, as was note above, sensor controller 28 and user interface controller 30 could be integrated into a single physical location, if desired.

Sensor controller 28 of FIG. 4 communicates with user interface controller 30, positioned inside of tablet 44, by a wired communication line 46. As shown in FIG. 4, wired communication line 46 comprises a 10/100 Megabits/second Ethernet cable. It will be understood by those skilled in the art, however, that other types of communication lines may be used, such as different types of Ethernet standards and/or speeds, as well as completely different communication protocols (e.g. Controller Area Network, Firewire, Local Operating Network (LON), various fieldbuses, etc.), as well as serial communication lines, wireless communication such as ZigBee or other IEEE 802 standard-based protocols, as well as other types of communication lines. Communication line 46 transmits bidirectional communication between flexible mat 32 and tablet 44. In this manner, tablet 44 is able to not only receive data from flexible mat 32, it is also possible for a user to issue commands via tablet 44, or other means, that forward commands or control signals to flexible mat 32.

In the embodiment illustrated in FIG. 4, user interface 26 is comprised of tablet 44, which includes a display or display screen 48. Display screen 48 may be a touch screen that senses the touch of a caregiver against display screen 48 and responds appropriately, depending upon the position on screen 48 which the caregiver touched, as well as the content displayed on screen 48 at the time the caregiver touched screen 48. In addition to, or in lieu of, a touch screen type of display 48, one or more keys, buttons, switches, computer mice, or other input devices could be coupled to tablet 44 to allow a caregiver to input data or control instructions.

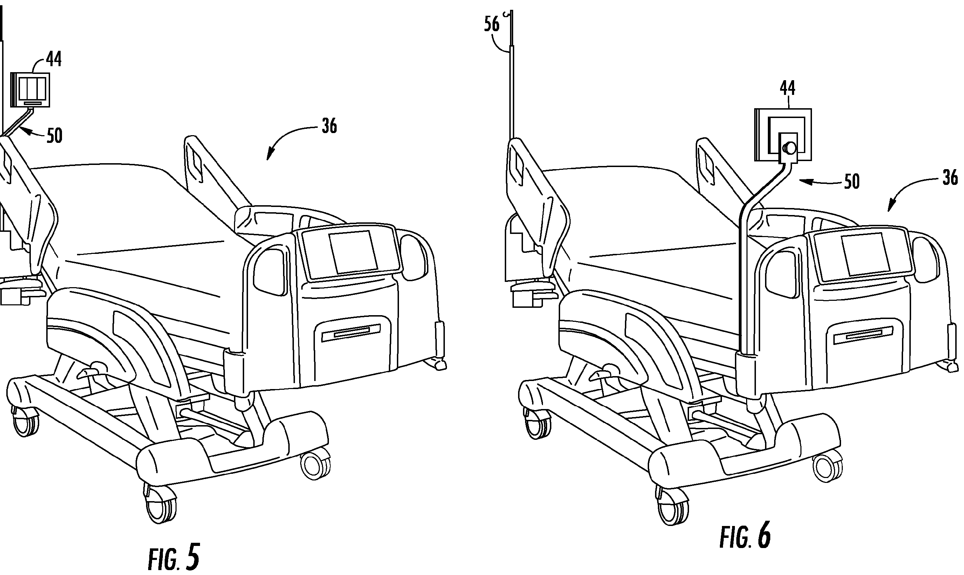

Tablet 44 in FIG. 4 is supported on a pedestal 50 that includes a boom arm 52 and a bracket 54. Pedestal 50 is configured to be mounted on patient support 36. Specifically, as will be discussed in more detail below, pedestal 50 is designed to be mounted to any of the conventional IV pole support holes defined on patient support 36. Typically, conventional patient supports 36 include an IV pole support hole at each corner of the patient support 36. FIGS. 5-6 illustrate a pedestal 50 mounted to two different IV pole support holes. In FIG. 5, pedestal 50 is mounted to an IV pole support hole positioned adjacent a first corner at the head end of the patient support 36. In FIG. 6, pedestal 50 is mounted to an IV pole support hole positioned adjacent a second corner at the foot end of the patient support 36. In both FIGS. 5 and 6, an IV pole 56 is shown mounted to the patient support 36. As will be discussed in greater detail below, pedestal 50 may be configured to support both itself and an conventional IV pole in the same IV pole support hole. In this manner, both pedestal 50 and an IV pole 56 may be simultaneously mounted in the same IV pole support hole of a patient support, such as is shown in FIG. 5.

In addition to a user interface controller 30, tablet 44 may include any one or more of the following features: a display screen; one or more light emitting diodes (LEDs); a removable memory-such as a flash memory card slot (e.g. Compact Flash, Secure Digital (SD), Memory Stick, xD, Multimedia Card (MMC), etc.)-or any other type of removable memory; a data processor; a hard reset button; a bar code or other type of light reader; a speaker and/or a buzzer; power and data connectors; swiveling and tiltable bracket; one or more batteries for providing backup electrical power in the case of a power supply disruption or during transport of a patient; and a wireless communication transceiver for enabling wireless communications between tablet 44 and a physically separate device, such as, but not limited to, a hospital communication network.

When configured with a wireless communication transceiver, tablet 44 may communicate with a hospital network such that information gathered from sensing system 20 can be forwarded to one or more other entities within the hospital, or other medical facility, that can make use of such information. For example, as will be discussed in more detail below, sensing system 20 can be configured to generate a variety of different alerts, depending upon how sensing system 20 is designed, and also depending upon how a user configures the various settings of sensing system 20. Such alerts may be desirably forwarded to a nurse call system within a medical care facility. By sending an alert wirelessly from tablet 44 to the hospital communication network, a server or other computer device associated with the nurse call system can detect the alert and forward it to the appropriate caregiver in a manner dictated by the nurse call system. Thus, system 20 can be connected to a nurse call system for forwarding alert conditions to a patient's caregiver.

As another example, system 20 can forward information to a hospital network that is picked up by an electronic medical records (EMR) server, or other device, in communication with the network. The electronic medical records server takes the data transmitted by tablet 44 and stores it in a particular patient's electronic medical records, thereby eliminating the potential for medical transcription errors, as well as recordation omissions. Tablet 44 may also communicate with other systems or subsystems that are communicatively coupled to the hospital network, besides nurse call system and an electronic medical system.

In addition to sending signals to a hospital communication network, tablet 44 may receive signals from the communication network that originate from a variety of different sources. For example, if a speaker and microphone are included on the tablet, voice communication between a remotely located caregiver and the patient may be effectuated using the wireless transmission of voice packets. As another example, a hospital's existing Admission, Discharge, and Transfer (ADT) system, which stores patient information, may communicate the name and other appropriate information to tablet 44 so that tablet 44 will know what patient is currently occupying the patient support that is being used with sensing system 20. Tablet 44 may then display this information to a caregiver, or use it to correlate medical records information so that information transmitted by tablet 44 to an EMR system can be correlated to a specific patient, or both.

While the use of a wireless transceiver in sensing system 20 has been discussed herein as being integrated into tablet 44, it will be understood by those skilled in the art that the wireless transceiver could be positioned in any desired location. Further, it will be understood that the specific manner in which the wireless communications take place can be varied. For example, the wireless communication may take place in accordance with any of the various IEEE 802.11 standards (802.11(a), (b), (g), (2007), (n)), or any other type of wireless communication standard. The hospital network may be an Ethernet, or other type of network, and the wireless transceiver of sensing system 20 may communicate with one or more wireless access points positioned within the hospital.

It will also be understood that, in at least some embodiments, sensing system 20 could communicate information to a hospital network using one or more wired connections. Such wired connections could utilize existing nurse call cables, or it could utilize wired connections to medical device hubs positioned within a patient's room, such as, for example, a CareAware Connectivity Engine marketed by Cerner Corporation of Kansas City, Missouri. Still other types of wired connections may be used.

Electrical power may be supplied to sensing system 20 in any suitable manner. As shown in the embodiment of FIG. 4, an electrical power cord 58 is included that plugs into a conventional electrical wall outlet. Power cord 58 is connected to an electrical transformer 60 that converts the AC wall outlet power into a DC current of suitable voltage for both sensor array 22 and tablet 44. Such DC power is carried to sensor array 22 and tablet 44 by wires 62 and 64, respectively. In alternative embodiments, sensing system 20 could be configured to draw power by tapping directly into a power source on the patient support 36. Still further, in at least one embodiment, sensing system 20 could receive power from patient support 36 through an inductive power transfer device positioned on the support 36 that inductively transferred electrical power from the frame of support 36 to sensing system 20 (as well as, in some cases, to a mattress), one example of such a system being disclosed in commonly assigned, co-pending U.S. patent application Ser. No. 13/296,656, filed Nov. 15, 2011 by applicants Guy Lemire, et al. and entitled PATIENT SUPPORT WITH WIRELESS DATA AND/OR ENERGY TRANSFER. Still further, patient support 36 itself could receive electrical power inductively from a wall or floor mounted inductive transfer station, examples of which are also disclosed in this commonly assigned 13/296,656 application, which is incorporated herein. A battery 66 could also be provided within tablet 44 for providing power during times when power from a wall outlet is not available, such as during transit of patient support 36, or at other times. Similarly, one or more batteries could be provided, if desired, for providing power to flexible mat 32 during times when electrical power is not available from a wall outlet.

In the embodiment illustrated in FIG. 4, tablet 44 also communicates with a patient support locator device 68 that is positioned on patient support 36. Such communication may take place over a communication line 72 that connects device 68 to table 44. The communication of tablet 44 with locator device 68 is an optional feature that may or may not be part of the designed capabilities of tablet 44. Patient support locator device 68 may be any type of device that is capable of being used to determine the location, such as the room number, of patient support 36. In some instances, where multiple patient supports 36 may be positioned within a single hospital room, locator device 68 may be configured to determine which section of the room the patient support is currently located.

In one embodiment, locator devices 68 may be configured in any of the manners described in commonly assigned, U.S. Pat. No. 7,598,853 issued to Becker et al., and entitled LOCATION DETECTION SYSTEM FOR A PATIENT HANDLING DEVICE. Locator devices 68 communicate with stationary locator devices 70 that are mounted in fixed locations, such as to a wall, ceiling, or other non-mobile locations. Stationary locator devices 70 transmit a signal that is unique to each device 70 and which can be detected by a device 68 when the device 68 is within relatively close proximity to a device 70. By storing a list of where each unique stationary device 70 is located within a hospital, or other facility, the location of a device 68-and also the patient support 36 to which is it physically coupled-can be determined by correlating the unique signal detected by device 68 (which was transmitted from a device 70) to the stored list that identifies the specific location for that particular device 70. Such correlation may be performed by electronic circuitry contained within device 68, or within tablet 44, or within other circuitry positioned on patient support 36, or by circuitry positioned remotely from patient support 36. If performed remotely from patient support 36, tablet 44 may wirelessly transmit the unique code or signal detected by patient support locator device 68 to a computer, server, or other remote component which then determines where patient support 36 is located by consulting the stored list of the locations of stationary devices 70.

Regardless of the precise manner by which bed locator device 68 determines the location of patient support 36, sensing system 20 may use this location information in multiple ways. By knowing either the actual room location of patient support, or information sufficient to enable a remote device to determine the actual room location, tablet 44 can include this information when issuing an alert. As noted previously, such alerts may be issued wirelessly via a WiFi connection, or any other wireless communication protocol. By including the room location in the alert, or information sufficient to enable the room location to be determined by a remote electronic component, the ultimate delivery of the alert message to the caregiver can include the location of the sensing system 20 that is issuing the alert.

Tablet 44 may also use room location data when transmitting data to an electronic medical record, or to an intermediate device or system that forwards the data to an electronic medical record. The transmission of room location data may be used by the electronic medical record system to correlate the transmitted data to a particular patient. In addition, or alternatively, the transmission of room location data may be used by the electronic medical records system as a cross check to insure that the transmitted data is being input into the correct patient's electronic medical record. For example, if tablet 44 transmits medical data and location information identifying its location as room 3446, the electronic medical record system may check one or more separate databases (such as, but not limited to, an Admission, Discharge, and Transfer-ADT-database) that correlate a specific patient and his or her room number, and if that room number does not match room 3446, an error signal may be communicated to the appropriate hospital personnel.

Tablet 44 may also use room location data in order to automatically populate its memory with all or any of the relevant patient data that it uses and/or displays. For example, once tablet 44 knows its room location, or sufficient information to determine its room location, it may transmit that information to a computer, server, or other remote device. In response, the computer, server, or other remote device may wirelessly transmit data to tablet 44 indicating who the particular patient is that is currently occupying patient support 36. Tablet 44 may then display all of some of this information on display screen 48 so that a caregiver will have access to this patient information. This also frees the caregiver from having to manually enter patient data into tablet 44. The patient information transmitted to tablet 44 may include any relevant patient information, including not only the patient's name, but also any and all medical information about the patient that may be desirably displayed on tablet 44, or otherwise used in conjunction with the operations performed by tablet 44 and/or sensing system 20.

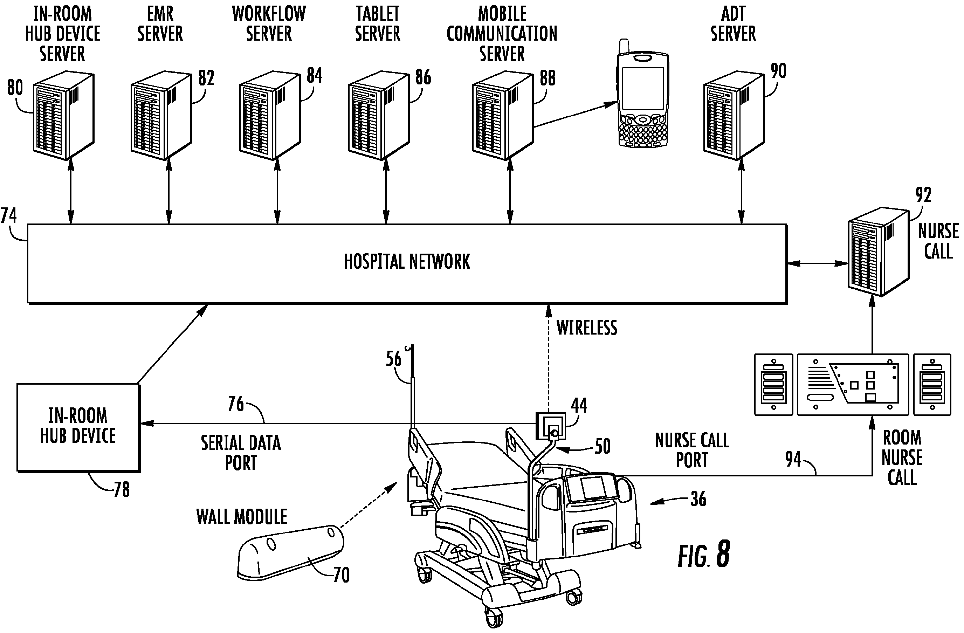

The manner in which alerts are forwarded to caregivers from tablet 44, as well as the manner in which patient data and/or other data is communicated to and from tablet 44, can vary. FIG. 8 illustrates a system diagram of one example of a hospital's computer network arrangement and the manner in which sensing system 20 may be integrated therein. More particularly, FIG. 8 shows two different manners in which system 20 may be integrated into a hospital's communication system. In a first manner, tablet 44 may communicate wirelessly directly with a hospital's computer network 74. Such communication may take place through one or more wireless access points (not shown) positioned throughout the hospital. In a second manner, communication may take place via a serial cable 76 that plugs into an in-room hub device 78. As was noted previously, one such in-room hub device 78 could be a CareAware Connectivity Engine marketed by Cerner Corporation of Kansas City, Missouri. Still other types of devices may be used. The in-room hub device 78 may act as a hub for receiving data cables from one or more other medical devices positioned within the room. The hub device 78 takes the data from each of the connected devices and forwards it the appropriate remote computer(s) and/or server(s) on the hospital network 74.

The hospital network 74 may be in communication with a plurality of different servers that each perform specific functions. For example, the hospital network 74 may be in communication with an in-room hub device server 80, an electronic medical records server 82, a workflow server 84, a tablet server 86, a mobile communications server 88, an admissions, discharge and transfer (ADT) server 90, and/or a nurse call server 92. The in-room hub device server 80, electronic medical records server 82, workflow server 84, mobile communications server 88, ADT server 90, and nurse call server 92 may be conventional servers or servers that are otherwise already part of the hospital's, or other healthcare facility's, information technology infrastructure.

The in-room hub device server 80 may function to process and route information transmitted over the hospital network from one or more in-room hub devices 78. The EMR server 82 may act as the electronic gateway for disseminating information from a patient's electronic medical records to any appropriate devices in communication with the hospital's computer network, as well as to receive and store information forwarded to it over the network 74 that is to be stored in a patient's electronic medical record. Workflow server 84 may oversee caregiver assignments, shifts, and other tasks associated with the caregivers.

Tablet server 86 may be included to receive and process information transmitted from one or more tablets 44 positioned throughout the hospital. When so included, tablet server 86 may forward information received from one or more tablets 44 to any of the other servers or other devices that are in communication with computer network 74. Tablet server 86 may also disseminate information to one or more tablets 44 that it receives from any of the servers or other devices that are in communication with computer network 74. In an alternative embodiment, tablets 44 could be configured so that they could talk directly to one or more of the non-tablet servers, thereby eliminating the need for a tablet server 86.

Mobile communication server 88 may be in communication with hospital network 74 so that information from any of the other servers, including tablet server 86 and/or tablets 44, may be forwarded onto to wireless, portable communication devices that are carried by caregivers. Such communication devices may include personal digital assistants, pagers, cell phones, smart phones, BlackBerries®, computer tablets, or other similar type devices. If sensing system 20 detects a condition that gives rise to an alert, tablet 44 may communicate this information to network 74-via either a wireless connection or a wired connection-where tablet server 86 may pick up this information and forward it to mobile communication server 88 for wireless forwarding to the appropriate personnel. Alternatively, as noted, tablet 44 may communicate this information directly to mobile communication server 88 in some embodiments.

ADT server 90 may also be in communication with hospital network 74 so that patient information stored in the ADT system may be disseminated to any device in communication with hospital network 74, including, but not limited to, one or more tablets 44. Still further, a nurse call server 92 may be in communication with network 74 so that information forwarded to the network from one or more tablets 44 may be communicated via the hospital's existing nurse call system, if desired.

As shown in FIG. 8, a conventional nurse call cable 94 may be coupled between patient support 36 and a conventional nurse call system. Nurse call cable 94 communicates data from patient support 36, as well as voice signals from a microphone that may be positioned on patient support 36. In at least one embodiment, tablet 44 does not use nurse call cable 94 for communications. Instead, as noted, tablet 44 may communicate either wirelessly or via a serial cable.

The foregoing description of sensing system 20 has provided a general overview of various of its components according to several embodiments. A more detailed description of several of these components are provided below. Specifically, each of the following components or aspects are discussed in greater detail below: flexible mat 32, controller 24, user interface 26, and additional sensors.

### Flexible Mat 32

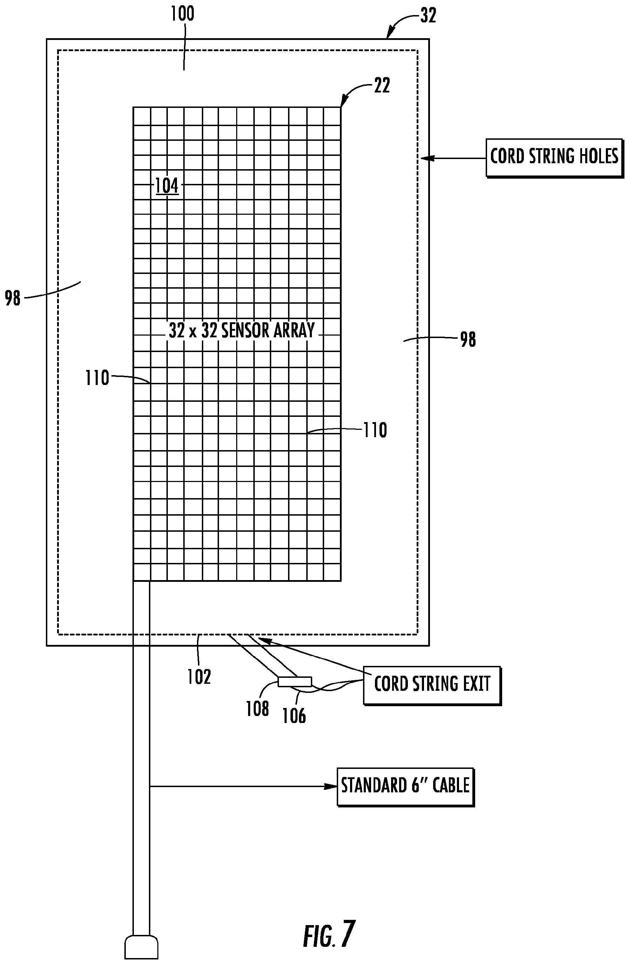

One embodiment of a flexible mat 32 into which sensor array 22 may be incorporated is shown in more detail in FIG. 7. Flexible mat 32 includes a pair of sides 98, a head end 100, a foot end 102, and a top surface 104. Flexible mat 32 may be made from a variety of different types of materials. In one embodiment, flexible mat 32 may be made from materials that have characteristics similar to a conventional fitted sheet that can be placed over a mattress. That is, flexible mat 32 may be soft and stretchy such that it does not substantially alter the feel of the underlying mattress to the patient. In some embodiments, flexible mat 32 and sensor array 22 may be made of materials that are X-ray compatible such that X-rays may be taken of a patient while in contact with mat 32 without having any of the materials of mat 32 interfere with the quality of the resulting X-ray image.

In the embodiment shown in FIG. 7, flexible mat 32 is adapted to be releasably coupled to a mattress, or other type of support surface, positioned on patient support 36 by way of a drawstring 106. Drawstring 106 extends into and out of the material of flexible mat 32 generally around the perimeter of mat 32 in the lower regions of sides 98, head end 100, and foot end 102. The location of drawstring 106 on flexible mat 32 is such that, when mat 32 is coupled to a mattress 38, drawstring 106 will be positioned underneath the bottom surface of the mattress. Thus, when drawstring 106 is pulled tight, the length of the perimeter defined by drawstring 106 will be reduced to a magnitude that is less than the perimeter of the mattress, thereby preventing flexible mat 32 from being removed from the mattress 38 without loosening drawstring 106. When drawstring 106 is tightened, it may be held in the tightened state by any conventional means, such as a clamp 108, or other type of locking mechanism that maintains the desired tension in drawstring 106.

Alternative mechanisms may be used to releasably secure flexible mat 32 to mattress 38. As but one example, suitably positioned Velcro may be incorporated into mat 32 that releasably attaches to correspondingly positioned Velcro on mattress 38, patient support 36, or other structure. In other embodiments, an elastic drawstring, or elastic structure similar to a fitted sheet may be used. In some embodiments, the mechanism used to secure mat 32 to mattress 38 will provide sufficient tension in mat 32 such that no portions of top surface 104 are likely to be creased, folded over, or otherwise bunched up in a manner that might interfere with the sensing of data by sensor array 22.

Sensor array 22 is incorporated into flexible mat 32 in the embodiment shown in FIG. 7. Sensor array 22 may take on a variety of different dimensions. As one example, sensor array 22 may have a width W of approximately 57 inches and a length L of approximately 75 inches. It will be understood by those skilled in the art, however, that this is but one of many possible sets of dimensions and that widths and lengths of different dimensions may be used. In the embodiment of FIG. 7 the dimensions of sensor array 22 are generally large enough such that sensor array 22 can detect patient pressure at substantially all locations on the top surface of mattress 38. That is, the dimensions of sensor array 22 generally match the dimensions of the top surface of mattress 38 so that sensor array 22 can detect patient pressure regardless of whether the patient's body or limbs are positioned along the sides of the support surface, or near the foot or head ends of the support surface, or anywhere in between these extremes. This allows sensing system 20 to detect patient pressure regardless of where the patient is positioned on mattress 38 of patient support 36.

It will be understood by those skilled in the art that sensor array 22 may be changed from the configuration shown in FIG. 7. For example, in some embodiments, it may be desirable for sensor array 22 to be positioned only over a portion of the top surface of mattress 38. Sensor array 22 can therefore be targeted to a specific area or zone of a patient's body in some instances. In the embodiment shown in FIG. 7, sensor array 22 is generally continuous. That is, there are no substantially gaps between individual sensor cells 110. In other embodiments, sensor array 22 may be configured such that gaps exist between the individual sensor cells.

The construction of sensor array 22 may be accomplished in a variety of different manners. In at least one embodiment, sensor array 22 is made of a plurality of row conductors positioned in a first plane, a plurality of column conductors positioned in a different plane, and a layer of piezoresistive material positioned in a third plane that is sandwiched between the first and second planes. The electrical resistance between a specific row conductor and a specific column conductor will thus depend upon the electrical resistance of the piezoresistive material sandwiched therebetween. By measuring this resistance, the pressure exerted by a patient on the portion of flexible mat 32 at the intersection of the specific row conductor and column conductor can be determined. By measuring this for all of the intersections of row and column conductors, the pressure exerted by the patient on mat 32 can be determined over the entire surface area defined by sensor array 32. Further, by repetitively measuring the pressures at each intersection, the pressure exerted by the patient on mat 32 over a time interval can be determined. A memory in communication with one of sensor controller 28, user interface controller 30, or some other suitable controller, may store the pressure readings, or a subset of the pressure readings, for various types of analysis, as will be discussed in more detail below.

It will be understood by those skilled in the art that other types of pressure sensing technology may be utilized other than piezoresistive technology. For example, sensor array 22, in at least one embodiment, could use a layer of piezoelectric material that generated voltage changes in response to patient pressure. As yet another alternative, sensor array 22 could be made of capacitive sensors that change capacitance in response to patient pressure. In still other embodiments, still other types of patient pressure sensing technology could be used in sensor array 22, including combinations of multiple different sensing technologies within the same array 22, or within the same mat 32.

In one embodiment, the physical construction of sensor array 22 may be the same as that disclosed in commonly assigned, co-pending, U.S. patent application Ser. No. 12/075,937 filed March 15,2008 by inventor Geoffrey L. Taylor and entitled ADAPTIVE CUSHION METHOD AND APPARATUS FOR MINIMIZING FORCE CONCENTRATIONS ON A HUMAN BODY. In another embodiment, the construction of sensor array 22 may be the same as that disclosed in commonly assigned, co-pending U.S. patent application Ser. No. 12/380,845 filed March 5, 2009 by inventor Geoffrey L. Taylor and entitled ELASTICALLY STRETCHABLE FABRIC FORCE SENSOR ARRAYS AND METHODS OF MAKING. In still other embodiments, the construction of sensor array 22 may be the same as any of those disclosed in commonly assigned U.S. Pat. Nos. 7,282,654 entitled PATIENT WEIGHING SYSTEM; 6,396,004 entitled PATIENT WEIGHING APPARATUS; 6,180,893 entitled PATIENT WEIGHING APPARATUS; or U.S. patent application Ser. No. 12/154,559 filed May 23, 2008 by inventors Peter Salgo et al. and entitled SYSTEM AND METHOD FOR PATIENT MONITORING. In yet other embodiments, sensor array 22 may be constructed in accordance with any of the sensing arrays disclosed in U.S. Pat. Nos. 7,201,063 entitled NORMAL FORCE GRADIENT/SHEAR FORCE SENSORS AND METHOD OF MEASURING INTERNAL BIOLOGICAL TISSUE STRESS; 6,543,299 ENTITLED PRESSURE MEASUREMENT SENSOR WITH PIEZORESISTIVE THREAD LATTICE; or 6,155,120 entitled PIEZORESISTIVE FOOT PRESSURE MEASUREMENT METHOD AND APPARATUS. In still other embodiments, the construction of sensor array 22 may combine any one or more of the features in any one of these incorporated references into a single array 22.

The size of sensor array 22 may be varied to cover areas significantly less than the surface area of the top surface of mattress 38, as well as areas significantly more than the surface area of the top surface of mattress 38. For example, sensor array 22 may be dimensioned to take pressure measurements over only a portion of the top surface of mattress 38. In other embodiments, the area of sensor array 22 may be larger than the top surface of mattress 38 such that sensor array 22 can detect pressure exerted by the patient against neighboring objects, such as a footboard, headboard, side rail, or other parts of patient support 36. Still further, in some embodiment, multiple separate sensor arrays 22 may be incorporated into one or more flexible mats 32 so that pressure readings from may be taken from different areas. In such cases, the flexible mats 32 may be positioned around different objects. For example, one mat 32 might cover mattress 38, while another mat 32 covered a pillow, while perhaps one or more additional mats covered patient positioners used to help turn a patient. Still more mats 32 with additional sensor arrays 22 could be used.

### Sensor Controller 28

Regardless of the number of sensor arrays 22 used in conjunction with a particular patient support 36, and regardless of whether the sensors arrays are incorporated into one or more flexible mats, the outputs from each sensor cell 110 within the sensor array may be detected by sensor controller 28. Sensor controller 28 may be configured to scan the outputs from each of the multiple sensor cells 110; that is, sensor controller 28 may be configured to sequentially take readings from each of the sensor cells. The particular order in which the outputs from the individual cells 110 is read by controller 28 may be varied. In some embodiments, controller 28 may include circuitry that enables it to simultaneously take pressure readings from two or more pressure cells, therefore reducing the overall amount of time it takes to take readings from all of the cells 110.

In some embodiments, sensor controller 28 will take measurements from each sensor cell 110 multiple times a second, such as, but not limited to, 1-10 times per second, or at other frequencies. In other embodiments, sensor controller 28 will take pressure measurements of a subset of sensor cells 110 at one or more different frequencies than one or more other subsets of different sensor cells 110. For example, if sensing system 20 is being used to detect a vital sign of a patient, such as, for example, a heart rate, then sensor controller 28 might be configured to take readings from those sensor cells 110 near the torso of the patient at a frequency that is higher than the frequency of readings taken from other sensor cells 110. The frequency at which sensor cell readings are taken may therefore be adjusted for different regions or zones of sensor array 22. Further, as will be discussed in greater detail below, the specific zones or regions may have boundaries that can be configured by the user, or which can be automatically configured by the system 20, and which may dynamically change as the patient moves. Still further, system 20 may automatically change the frequency at which readings are taken from specific zones depending upon what the zone is, its relationship to the patient's body, and/or any other desired factors.

Sensor controller 28 may take pressure readings from each individual pressure cell 110 in any of a variety of different manners. If sensor array 22 is comprised of piezoresistive materials whose electrical resistance varies in response to pressure, any circuitry capable of detecting changes in the electrical resistance level within cells 110 may be used. Such circuitry, for example, might detect changes in electrical current, changes in voltage, changes in capacitive or inductive time constants, or any other electrical characteristics that are influenced in a known manner by changes in the electrical resistance of cells 110. Such types of circuitry would be within the skill of one of ordinary skill in the art. If sensor array 22 uses sensing technology other than piezoresistive materials, such as piezoelectric, capacitive, or other technology, sensor controller 28 may detect the changes using still different types of circuitry.

In converting the measured electrical characteristic, such as resistance, into a pressure reading, sensor controller 28 may rely upon one or more calibration measurements. Such calibration measurements may be performed in any manner. As one example, calibration may be performed by placing known amounts of weight and/or pressure over the sensor array 22 and detecting the readings from each of the cells 110 that are experiencing a force component from the weight and/or pressure. Calibration values can then be determined for future use in correlating the electrical readings from the sensor cells 110 to pressure values. The pressure values may be measured in any suitable units (such as, but not limited to, inches of mercury, pascals, bars, etc.).

Depending upon the construction of sensor array 22, calibration may be repeated at various times throughout the life of sensor array 22 and/or flexible mat 32. Whether such calibration is repeated or not, the results of the calibration may be stored within a memory accessible to sensor controller 28. When sensor array 22 is then in use, sensor controller 28 can read the calibration value or values from the memory and use in the conversion of raw electrical readings into pressures. In one embodiment, the memory may be a non-volatile memory that is contained within a physical housing that also houses sensor controller 28. For example, a memory may be contained with the sensor controller 28 (marked ECM) shown in FIG. 4. By storing these values here, a flexible mat 32 and its associated controller 28 can be replaced on a particular patient support without changing the user interface, and/or any of the other components of sensing system 20. In other words, the ECM can carry out all of the calibration functions without having to rely on any separate or extraneous components to do so, thereby freeing the user interface 26 and/or user interface controller 30 from being involved in the calibration process. In other embodiments, however, one or more aspects of the calibration process may be performed by user interface controller 30, or some other controller other than sensor controller 28.

In some embodiments, sensing system 20 may be configured to carry out an automatic, or semi-automatic, calibration process by using data from one or more weight sensors that are integrated into patient support 36. Many conventional patient supports include a scale system, which may include load cells or other sensors, that enable the patient support to determine the weight of a patient, or other items, positioned on the patient support 36. Sensing system 20 may be configured to receive this weight data and use it to automatically calibrate a particular sensor array 22. The manner in which sensing system 20 receives this weight data may vary. For example, patient support 36 may include a data port built into it that allows a data cable to be connected between one or more components of sensing system 20 and an electronic interface of patient support 36 that outputs the weight data sensing by the patient support's scale system. Thus, for example, if the scale system of patient support 36 indicates that a weight of 150 pounds is currently positioned on top of the support surface of the patient support, this data can be communicated to sensing system 20 so that one of the controllers within system 20 can correlate the sensing weight to the sensor cells readings. The particular component of sensing system 20 to which the weight data may be communicated can be varied, but could include sensor controller 28, or user interface controller 30, or some other component.

If sensing system 20 is configured to use weight data from patient support 36 for calibration purposes, sensing system 20 may perform such calibrations automatically at any time that valid weight data is available from the bed, or it may perform it automatically at certain configurable intervals (e.g. daily, weekly, monthly, every time a new patient uses the system, etc). Alternatively, system 20 could be configured such that an auto-calibration is only performed in response to a user pressing a specific button, or icon, or the like, on user interface 26. As yet another alternative, system 20 could be configured to enable automatic periodic calibrations, as well as manually chosen times for carrying out the automatic calibrations.

In some embodiments, sensor controller 28 may be physically and removably separable from flexible mat 32. In such embodiments, if flexible mat 32 has to be cleaned or discarded, or the like, sensor controller 28 may be removed and used with another flexible mat. This decreases the overall cost of replacement mats 32. Such removability of ECM 28 may be accomplished in any of a variety of different manners. As one example, a pocket, or the like, may be sewn into a side section 40 (FIG. 4), or other portion, of flexible mat 32. Sensor controller 28 may then be physically inserted into the pocket and then electrically coupled to one or more cables that are in electrical communication with each of the sensor cells 110. Communication line 46 and/or power line 62 may then be coupled to sensor controller 28.

### Conditions Sensed

Sensing system 20 uses the outputs from sensor array 22 to detect one or more different conditions that relate to the patient and/or the patient support 36. For many of these conditions, the sensing is performed by performing one or more algorithms on the pressure data that is gathered from sensor cells 110. The processing of these algorithms may take place in any suitable controller. That is, the processing may be performed by sensor controller 28, user interface controller 30, both, or either wholly or partially by still other controllers. Some of the various conditions that may be sensed by sensing system 20 are discussed in greater detail below.

In one embodiment, sensing system 20 may detect whether a patient has sat up while being positioned on flexible mat 32, or on any other structure that incorporates one or more sensor arrays 22. The detection of a patient sitting up can be accomplished by analyzing the outputs of sensor array 22 in various manners. For example, a controller (such as controllers 28, 30, or otherwise) can look for changes in the total area over which a patient's pressure is being exerted on sensor array 22, combined with changes in pressure sensed by sensor array 22. Thus, as but one example, a controller can look for a threshold decrease in the area over which pressure is distributed along with a corresponding increase in the magnitude of the pressure within the area on which the patient is still in contact. This situation would typically occur when a patient moves from a supine position-in which their weight is distributed over a relatively large area-to a sitting up position, in which their weight is distributed over a smaller area (e.g. the patient's derriere), but with an increased magnitude. In other embodiment, only changes in the total area of sensor array 22 contacted by the patient may be used, without taking into account changes in the magnitude of the pressure. In still other embodiments, only changes in the magnitude of the pressure may be analyzed to determine a patient sitting up, without taking into account the size of the area over which the pressure is distributed.

Still other manners in which the transition to sitting up can be detected may be used. In one such embodiment, the controller may look at changes in specific zones on the sensor array 22 that are experiencing pressure. If the patient's weight distribution changes to become more focused on a particular zone, such as a zone corresponding to the patient's posterior, then this can be used to indicate that a patient has sat up. The use of zones is described in more detail below.

A user of sensing system 20 can determine whether an alert should be issued when a patient sits up. This can be done via user interface 26. If an alert is not desired, user interface can instead, if desired, simply record the time at which the patient was determined to have sat up. This time may then be retrieved via user interface 26 for later viewing by caregivers. Still further, if a user desires, he or she can utilize user interface 26 to electronically forward information about the act of sitting up to an electronic medical record server or system via the wireless connection within tablet 44, or any other suitable electronic connection. System 20 may further record the pressure distribution sensed by sensor array 22 during the patient's transition to the sitting up position-as well as before and after, if desired-and this can be stored in tablet 44 for later viewing, as well as for forwarding to an electronic medical record.

Sensing system 20 may also be configured, in some embodiments, to detect shear pressure that is potentially being experienced by a patient, whether this is done in addition to, or in lieu of, the sensing of a patient sitting up, or any of the other various conditions that may be sensed by sensing system 20. Sensing system 20 may detect shear pressure conditions by analyzing the outputs of sensor cells, such as sensor cells 110, that only directly measure perpendicular force components directed thereagainst (i.e. compressive forces), or it may detect shear pressure by using specialized sensors that actually measure shear pressure.

If flexible mat 32 only includes sensors that detect compressive forces exerted thereagainst, the existence of undesirable shear pressure can be inferred from these sensors in multiple different manners. In one manner, either controller 28 or controller 30, or some other controller, analyzes the outputs of the sensor cells 110 and looks for sensor cells, or groups of sensor cells, that are experiencing relatively light amounts of pressure that are moving back and forth. This back and forth pressure will be caused by a patient moving a portion of his or her body back and forth. This back and forth motion of the patient against the mattress 38, or whatever other object the patient is resting on, can result in undesirable shearing forces exerted against the patient. Such shearing forces can lead to skin breakdown, or other undesirable results.

If flexible mat 32 only includes sensors that detect compressive forces exerted thereagainst, the existence of undesirable shear pressure can also, or alternatively, be inferred from these sensors by looking for regions of relatively large magnitudes of pressure moving relatively slowly and generally continuously in one or more directions parallel to the plane of the top surface of mattress 38 (i.e. either right or left across mattress 38, or up or down on mattress 38). Such movement of a patient's body, or portions of his or her body, can also create undesirable shear pressure because such movement is representative of a dragging motion across the mattress 38. The precise thresholds of the pressure magnitudes, rate of movement, and magnitude of movement that trigger a shear pressure alert may be static, or they may vary depending upon such things as, for example, the weight and/or size of the patient. They may also vary as a function of any one of the variables of the pressure magnitude, rate of movement, and magnitude of movement. In other words, if the moving region of pressure has a higher magnitude, the thresholds of the distance moved and/or the speed of the movement necessary for an alert may be decreased, or otherwise changed. Conversely, if the moving region of pressure has a lower magnitude, the thresholds of the distance moved and/or the speed of the movement necessary for an alert may be increased, or otherwise changed.

As will also be discussed in more detail below, the thresholds that generate a shear pressure alert may also be variably based upon a Braden scale that is assigned to a particular patient. The Braden scale assigns a number to a patient that indicates a patient's risk level for experiencing pressure sores. The Braden scale number for a particular patient may be input into system 20 by a caregiver using user interface 26, or it may be communicated from an EMR, or other source, wirelessly via tablet 44's wireless connection, or via some other route. Once system 20 has this number, it may adjust the thresholds that generate pressure alerts (including not just shear force alerts, but also compressive force alerts) in response thereto. Thus, for example, a patient at a higher risk of a pressure sore may generate an alert condition more easily than a patient having a lower risk of a pressure sore. In this manner, the pressure alerts can be tailored more specifically to the patient currently occupying patient support 36.

Still other manners of detecting undesirable shear conditions using compressive pressure sensors may also be used. If sensing system 20 is equipped with shear pressure sensors that directly measure shear pressure-and which may be incorporated into flexible mat 32-then the analysis of signals necessary to determine an undesirable shear pressure condition may be simpler.

Regardless of the manner used by sensing system 20 to detect an undesirable shear condition, sensing system 20 may issue an alert so that caregivers are aware of the undesirable situation. The alert may be transmitted wirelessly from tablet 44 to the hospital network 74 for forwarding to mobile communication server 88, which then forwards an alert to the mobile device of an appropriate caregiver. System 20 may alternatively be configured to issue an alert locally, i.e. within the vicinity of patient support 36. Such a local alert may be aural or visual, or a combination of both.

In addition to issuing an alert of an undesirable shear condition, sensing system 20 may also record the pressure changes detected by sensor cells 110 for later playback, or later viewing. This data may also be forwarded to an electronic medical record, or to a remote location for viewing by a caregiver.

Sensor array 22 may also be used to issue a bed exit alert. When so configured, a caregiver can convert a patient support 36 having no bed exit alerting capabilities into a patient support having bed exit alerting capabilities. Alternatively, sensor array 22 can be used on patient supports 36 that already have bed exit alerting capabilities. Such dual bed exit alerting capabilities may be desired for redundancy purposes, or because sensing system 20 may offer different or better bed exit sensing capabilities.

When configured to detect a patient exiting patient support 36, sensing system 20 may provide different types of alerts. For example, in one embodiment, an alert might be issued only when sensor array 22 ceases to detect any patient pressure (i.e. the patient is completely off patient support 36). In another embodiment, an alert may be issued while the patient is still on patient support 36, but the pressure has changed in such a manner as to indicate that a bed exit action by the patient may be likely. Such changes may involve the patient moving to a side region of the patient support, or moving to other regions, or moving in one or more particular other manners, as detected by sensor array 22. A bed exit alert may therefore be issued prior to the patient actually exiting the patient support. The level of movement by the patient, or the kind of movement by the patient, that triggers such an alert can be, in at least one embodiment, configured by a caregiver. The caregiver can therefore select the threshold levels of activity necessary to trigger an alert so as to provide the desired level of monitoring that matches the needs/conditions of the current patient in patient support 36.

Sensing system 20 can also be used to detect if a patient has bottomed out on the mattress 38. Bottoming out occurs when all or a portion of the top surface of mattress 38 is compressed by the patient to such an extent that it effectively reaches the surface underlying mattress 38. Stated alternatively, bottoming out occurs when the compressive pressure is such that the mattress cannot be squeezed to any greater practical extent, thereby draining the mattress 38 of providing any cushioning effect to the patient. Bottoming out can be detected by sensor cells 110 when the force detected by one or more sensor cells exceeds a threshold, or when groups of sensor cells collectively detect pressure levels above a threshold. In both cases, the threshold may be a function of the type of material that mattress 36 is made out of, as well as its thickness, and/or other factors. Such information may be communicated automatically to sensing system 20 via a data pathway between mattress 38 and any of the electrical components of sensing system 20. System 20 may then adjust the criteria used to detect bottoming out based upon the specific mattress 38 currently being used on patient support 36.

It will be understood that the use of the term "mattress" in the written description herein refers to both conventional mattresses, as well as patient support surfaces that include fluid bladders, and/or still other types of support surfaces. References to "mattress 38" therefore include fluid filled structures, conventional mattresses, and any other type of structures that provide cushioning support to a patient while positioned on patient support 36. One example of a fluid filled structure that may be used in any of the embodiments disclosed herein is shown in commonly assigned U.S. patent Pub. No. 2011/0301516 filed Feb. 7, 2011 by applicants Patrick Lafleche et al., and entitled PATIENT/INVALID HANDLING SUPPORT.

In yet another alternative embodiment, sensing system 20 may detect bottoming out of patient by using separate sensors (other than sensor cells 110) to detect such bottoming out. Still further, in some embodiments, sensing system 20 may use a combination of sensor cells 110 and other sensors to detect bottoming out. Such additional sensors may be any of a variety of different conventional sensors, as would be known to one of ordinary skill in the art. In some cases, such sensors may be integrated directly into flexible mat 32. In other embodiments, such sensors may be placed elsewhere. Such sensors may detect bottoming out by measuring the distance between the patient and the top surface of the support that underlies the mattress 38, or by measuring other quantities.

In some embodiments, one or more accelerometers may also be incorporated into flexible mat 32. Such accelerometers may be positioned in known orientations so that the angle of one or more regions of flexible mat 32 can be determined with respect to gravity. The accelerometers may be communicatively coupled to sensor controller 28, or another controller, that reads and processes their outputs. If multiple accelerometers are included, they may be oriented at different orientations so that different angles may be measured by the several accelerometers. One angle that may be measured by the accelerometers is what is known as the head of bed angle (HOB). HOB generally refers to the angle of inclination of the Fowler section of patient support 36. This information is often desirably monitored in certain situations to help prevent the occurrence of ventilator associated pneumonia, the patient's susceptibility to which can increase if they are lying flat, or at a non-elevated angle.

Regardless of the purpose for monitoring the HOB, however, a user of sensing system 20 can configure the system to issue an alert if the HOB decreases below a user-configurable threshold, or if it exceeds a user-configurable threshold. A history of the HOB angle may also be recorded and stored, either locally within system 20, or it may be forwarded to an EMR system via tablet 44's wireless connection to the hospital, or via another connection. As with any of the alerts that may be issued by sensing system 20, the alert may be local (audio or visual or both) and/or it may be remote.

Accelerometers may also be used to confirm that a patient has been turned an appropriate amount and/or for a sufficient amount of time. For some medical protocols, it is desirable to turn a patient for at least a minimum angular amount for a certain amount of time. In past systems, the angular amount of turning of the patient has been assumed. However, with sensing system 20, an actual measurement of the angular amount of rotation can be generated so that that caregiver(s) will know that the patient has been sufficiently turned. This information, as well as any information generated from sensor array 22, can be displayed on display screen 48 of user interface 26. It may also be stored, forwarded to an EMR, or used in any other desirable manner.

### Pressure Distribution Profiles

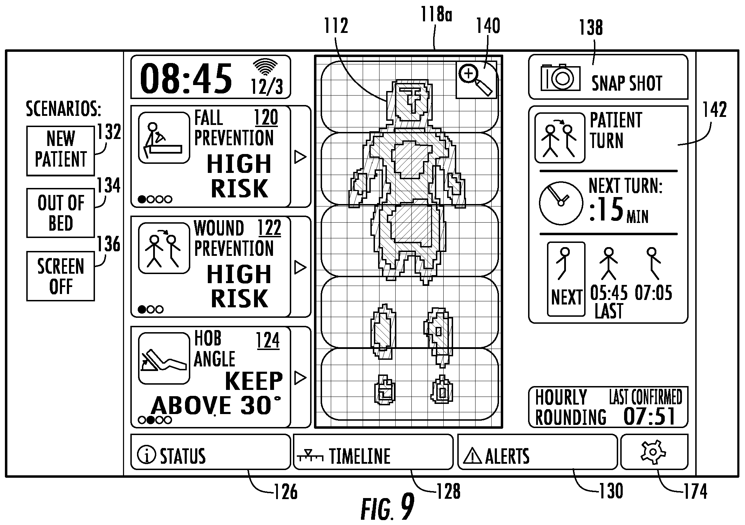

FIG. 9 illustrates one manner in which information may be displayed to a user of sensing system 20. More specifically, FIG. 9 illustrates an illustrative screen shot that may be displayed on a display in communication with, or integrated into, sensing system 20, such as, for example, display screen 48 of tablet 44. A pressure distribution profile 112 is shown in the example of FIG. 9 that is generated from the data gathered by pressure sensor cells 110. Pressure distribution profile 112 indicates graphically the locations and magnitudes of the pressure currently being detected by sensor array 22. The magnitudes of the pressure may be indicated according to a color scale wherein certain colors represent defined ranges of pressure. Shades of red may indicate undesirable levels of pressure, or other colors may be used.

In the example shown in FIG. 9, it is evident from the pressure distribution profile 112 that a patient is lying down on sensor array 22 (which may be incorporated into flexible mat 32). One or more of the controllers either integrated into sensing system 20 (e.g. sensor controller 28 or user interface controller 30), or in communication with sensing system 20, may be programmed to analyze the pressure distribution profile currently being detected by sensor array 22, or previously detected by sensor array 22, to map where a patient's body parts are located on mattress 38. By correlating the pressure readings to specific parts of the body, sensing system 20 may provide additional useful information to a caregiver, or EMR.

The manner in which sensing system 20 can correlate or map pressure readings to patient body parts can be varied. In one embodiment, software or firmware is included within a suitable controller that is programmed to analyze and recognize patterns from the shapes of the pressure readings being detected by sensor array 22. Such software or firmware may be similar to conventional image recognition software. Based upon the analysis of the shapes of the pressure readings, as well as the magnitude of the readings, the software or firmware can determine which body parts are exerting pressure against which sensor cells 110. In making this correlation, additional sensors may also be used, in some embodiments. A camera, for example, could be positioned on pedestal 50, or elsewhere, that occasionally takes digital pictures of the patient that are analyzed using image recognition software to determine the patient's body location on patient support 36. Such digital pictures may be used either by themselves to map the patient's body location to the sensor cells 110, or they may be used in combination with software that analyzes shapes, patterns, movement, and/or magnitudes of sensor cell readings so that both the digital images and the sensor cell readings are used to determine the patient's body location and/or orientation vis-à-vis the sensor cells 110.

In some embodiments, sensing system 20 may be configured to determine the boundaries, or assumed boundaries, of the patient's body and to display that as part of the pressure distribution profile 112. A boundary line 114, such as is shown in FIG. 9, may be displayed on display screen 48 showing the calculated boundaries of the patient's body on the patient support. This may be useful for conveying to the caregiver how the pressure distribution profile 112 maps to the patient. In other embodiments, a different manner of showing the correlation between the patient's body and the sensed pressures may be used. In still other embodiments, there may be no visual indications of the correlation between the patient's body and the pressure readings.

One of the many possible uses of determining a patient's body location and/or orientation is for issuing alerts that may be based upon the patient's body position and/or orientation. For example, with some patients, it may be desirable that the patient avoid turning on a particular side, due to any of a variety of different medical reasons. By determining the orientation of the patient on patient support 36, system 20 may issue an alert if the patient turns to the undesired orientation. Such alert settings may be configured by a caregiver via user interface 26.

### User Interface

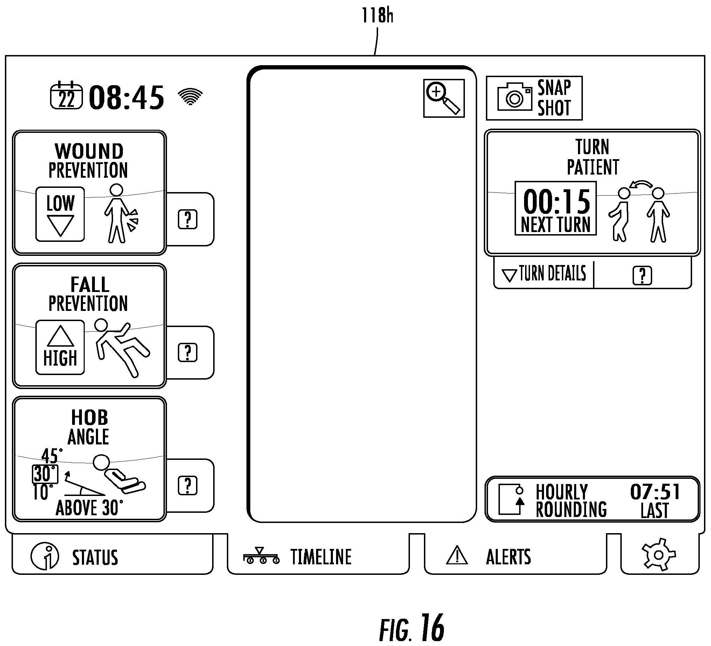

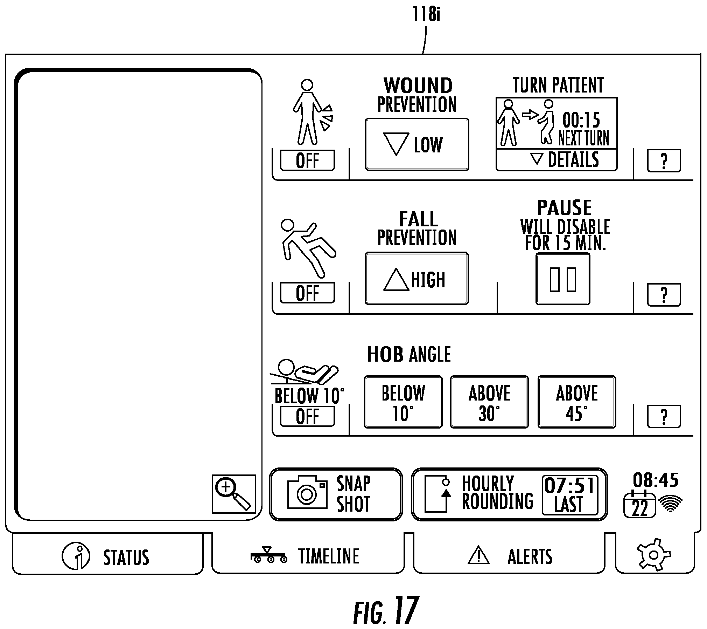

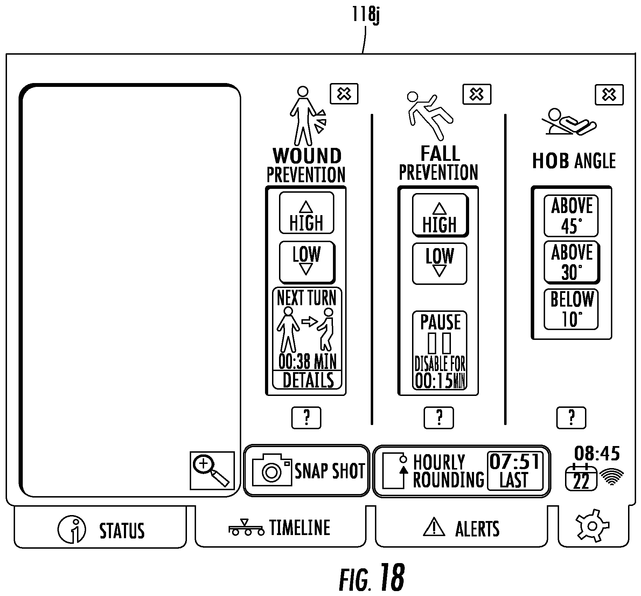

FIGS. 9-20 depict examples of illustrative screen shots 118a-I or screen layouts that may be displayed on display screen 48 of tablet 44 in various embodiments, or on another screen associated with sensing system 20. The screen shots of FIGS. 9-20 primarily illustrate aspects of sensing system 20 that relate to fall prevention, wound prevention (such as pressure ulcers), and head of bed (HOB) angle monitoring. In some embodiments, additional features may be monitored and/or controlled using user interface 26, beyond those illustrated in FIGS. 9-20. In other embodiments, sensing system 20 may have more limited features than what is shown in FIGS. 9-20.

The screen shots 118a-I of FIGS. 9-20 may be interactive. That is, if these screen shots are displayed on a touch screen display, then touching various of the images depicted therein will cause the screen to change in response thereto, generally in a user-intuitive manner. By touching the screen in the appropriate spots with the appropriate menus, icons, or other images, a caregiver can control various settings of sensing system 20. If a touch screen user interface is not used, control of sensing system 20 via these screen shots may alternatively be carried out using a computer mouse, a trackball, a light pen, a keypad, a track pad, or any other suitable control device that enables the user to interface with, and control, the various features of sensing system 20.

In the screen shot 118a of FIG. 9, a fall prevention control 120, a wound prevention control 122, and a HOB angle control 124 are shown. Along the bottom of the screen are shown additional controls, including a status control 126, a timeline control 128, and an alert control 130. Additional icons, images, windows, or other types of control are included in the screen shot 118a of FIG. 9, including a new patient control 132, an out of bed control 134, a screen off control 136, a snap shot control 138, a zoom control 140, and a patient turn control 142. Each of these controls respond to a user touching them-if they are displayed on a touch screen-or in an equivalent manner if a non-touch screen is used. In other words, if a non-touch screen is used to display these images, they will respond to a computer mouse clicking on them, for example, in the same manner as they would if a user pressed them on a touch screen. For purposes of description below, the various controls 120-142 of screen shots 118a-I will be described with respect to a touch screen. It will be understood, however, that the concepts and control features described are not limited to touch screens, but can be implemented using other types of user interface control mechanisms. Still further, it will be understood that the layouts shown in these screens shots 118a-I and the manner in which they respond to user inputs can be varied from that shown.

By selecting fall prevention control 120, a user can control the bed exit alerting capabilities of sensing system 20. If a patient is at a high risk for falls, the caregiver can select control 120. In response to each pressing (or other selecting) of control 120, the image corresponding to fall prevention control 120 will change, and the criteria for which sensing system 20 will issue a bed exit alert will change. Thus, in screen shot 118a, the "high risk" fall prevention setting has been chosen. With this setting, system 20 will issue a bed exit alert-as detected by sensor array 22-when a relatively minimal amount of a specified type of patient movement occurs. If the user presses control 120 again, the risk level will change to other levels of risk, such as, for example, "medium," "low," or "off." Sensing system 20 will respond to these changes by changing the criteria that cause it to issue a bed exit alert. In general, wound prevention control 122 and HOB control 124 work in similar manners. That is, selecting these buttons allows the caregiver to change the criteria that will cause an alert with respect to wound prevention criteria or HOB of bed angle criteria, respectively.

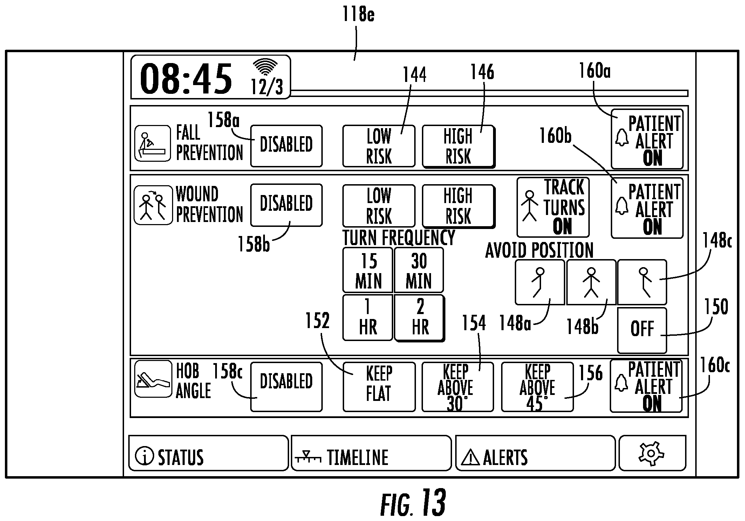

One example of the types of settings that a user may choose from when selecting any of the fall prevention, wound prevention, and HOB controls is illustrated in screen shot 118e of FIG. 13. In the embodiment shown therein, fall prevention control 120 only allows the caregiver to choose from three different settings: a "disabled" setting in which the bed exit alert is turned off; a "low risk" setting in which a bed exit alert is only issued when patient movement activity satisfies system 20's criteria for a likely bed exit; and a "high risk" setting in which a bed exit alert is issued when patient movement activity satisfies different criteria for a likely bed exit, wherein the "high risk" setting will cause an alert to issue sooner than the "low risk" setting, or will otherwise provide more suitable alerting for a patient who is at a high risk for falls.

With respect to the wound prevention controls, screen shot 118e of FIG. 13 shows that a level of risk can also be set by the caregiver using "low risk" icon 144 or "high risk" icon 146. The caregiver can also select how frequently the patient should be turned, which is a technique for helping to prevent pressure sores, or other types of wounds, for forming and/or becoming exacerbated. The settings for the wound prevention controls further include a set of "avoid position" icons 148a-c that, when one or more are selected, cause an alert to be issued from system 20 if the patient moves to one of these undesirable positions. Button 148a corresponds to a person lying on his or her right side; button 148b corresponds to a patient lying flat on his back; and button 148c corresponds to a person lying on his or her left side. Additional or fewer position buttons could also be included. An "off button 150 is also included to shut off the "avoid position" settings such that no alerts are issued based upon the patient moving to these positions. The wound prevention control settings shown in FIG. 118e of FIG. 13 further include a "track turns" button that allows the caregiver to set system 20 so that it will keep track of patient turns.

Screen shot 118e of FIG. 13 also illustrates several exemplary control settings that may be associated with the HOB angle control 124. As can be seen therein, several buttons are included that enable the caregiver to choose what type of angular conditions will cause an alert to be issued. For example, a "keep flat" button 152, when selected, will cause an alert to be issued by system 20 if the HOB angle-as measured by flexible mat 32 (via accelerometers, or other sensors)-moves from a flat orientation to a non-flat orientation. In a like manner, a "keep above 30 degrees" button 154 will, when selected, cause an alert to be issued by system 20 if the HOB angle changes to a value less than 30 degrees. Button 156 does the same thing for HOB angles less than 45 degrees.

Any one of a set of "disable" buttons 158a-c may be selected in order to completely shut off any of the fall prevention alerts, wound prevention alerts, and HOB alerts, respectively. A status icon, such as icons 160a-c may indicate to the caregiver which one of these alerts are on and which ones are off.

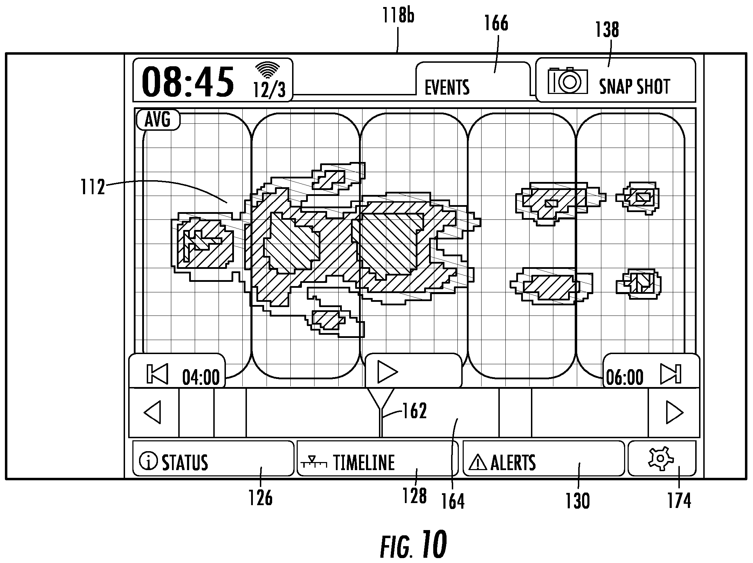

FIG. 10 illustrates an illustrative screen shot 118b that may appear when a caregiver selects the timeline control 128. Selecting the timeline control 128 will cause system 20 to display a pressure distribution profile 112 of the patient, as sensed by sensor array 22. Further, system 20 will display a mechanism, such as a time selector 162, for allowing the caregiver to view the changes in the pressure distribution profile 112 over time. With time selector 162, a caregiver can drag selector 162 left or right along a time line 164. Pressure distribution profile 112 will change to correspond to the pressure distribution profile that was sensed at the moment corresponding to the position of time selector 162. Once time selector 162 is moved to a desired position-in at least some embodiments-a "play" button may be included that, when selected, displays the pressure distribution profiles 112 from the selected moment in time onward, resulting in the image of the pressure distribution profile 112 to change in a movie-like fashion.

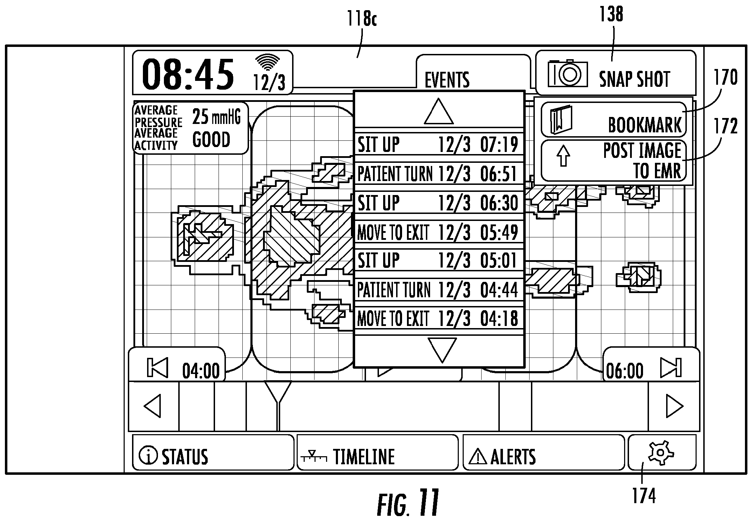

Screen shot 119b (FIG. 10) further includes an "events" button 166 and a "snap shot" button 138, either of which may be selected by a caregiver. FIG. 11 displays how the images in the screen shot may change in response to these buttons being selected. More specifically, as shown in FIG. 11, selecting the events button 166 will cause a listing of events detected by sensing system 20 to be displayed, including the time and date at which these events were detected. The events may include such things as the patient sitting up, the patient turning, the patient moving to exit, and any other patient condition that may be detected by sensing system 20. FIG. 11 also displays several options that may be presented to the caregiver after selecting the snapshot button 138. Specifically, it includes a "bookmark" option 170 and a "post image to EMR" option. By selecting the bookmark option, a virtual bookmark will be associated with the pressure distribution profile 112 that is being displayed at the time the bookmark 170 option is selected. This enables the caregiver to return to this "bookmarked" position easily for later reference. Multiple different instances in time of pressure distribution profile 112 may be bookmarked via button 170. Button 172, when selected, allows a caregiver to forward the pressure distribution profile 112 to an electronic medical record (EMR).

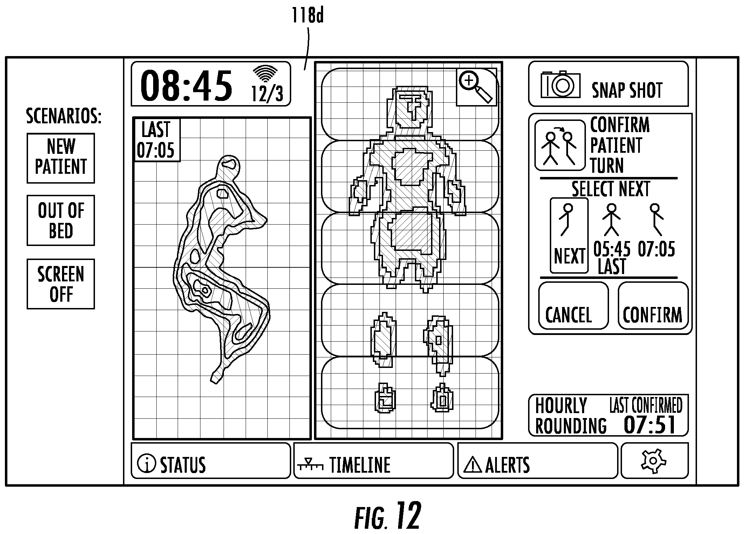

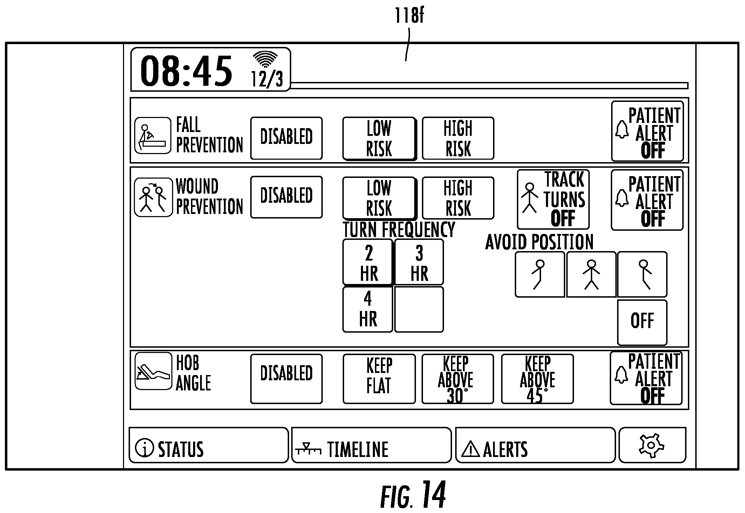

FIG. 12 shows an illustrative screen shot 118d that may be displayed in response to a user selecting the status control 126. FIG. 14 shows an illustrative screen shot 118f that depicts another manner in which information and control settings may be presented to a user of sensing system 20.

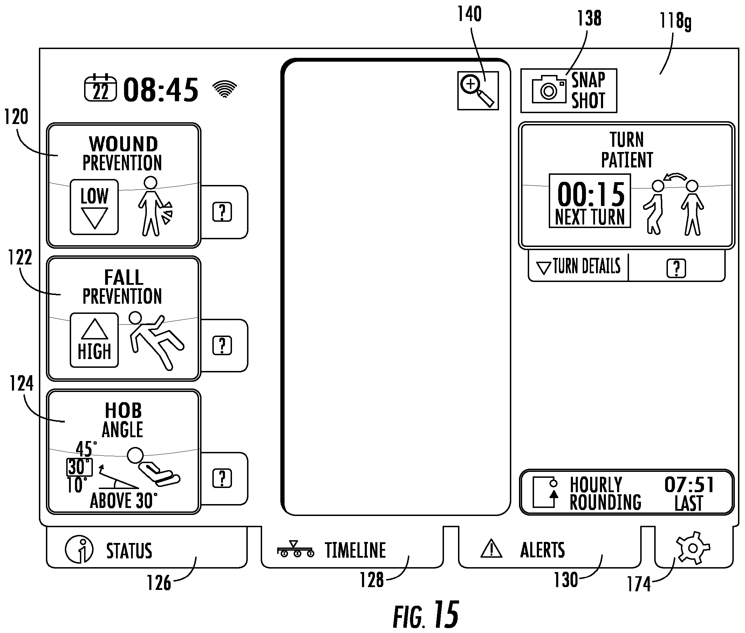

FIGS. 15-18 depict alternative manners in which information and control settings may be presented to a user of sensing system 20. In all of FIGS. 12-18, the various control settings and buttons operate in the same basic manner as has been described above.

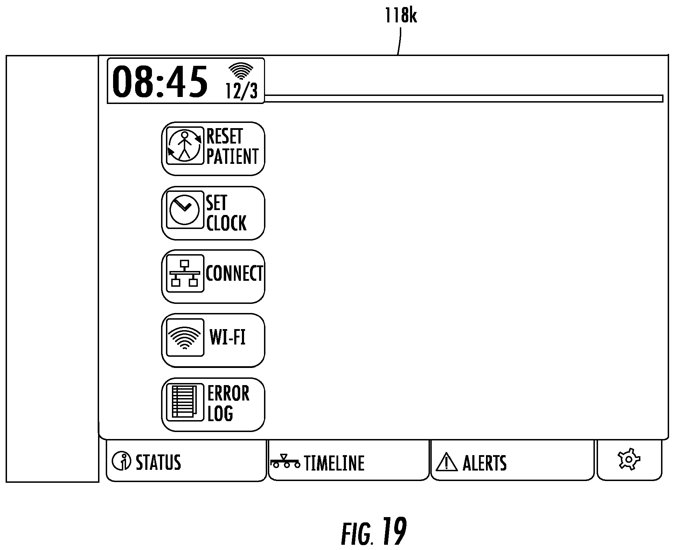

FIG. 19 shows an illustrative screen shot of the various features, and operational functions that a caregiver may control after selecting a button 174. Thus, this enables the caregiver to reset the information contained within sensing system 20 when a new patient is positioned on patient support 36. It also allows the caregiver to set a clock, connect to the hospital's network 74 (which may provide Internet access), control various of the WiFi settings, and/or see and manipulate an error log.

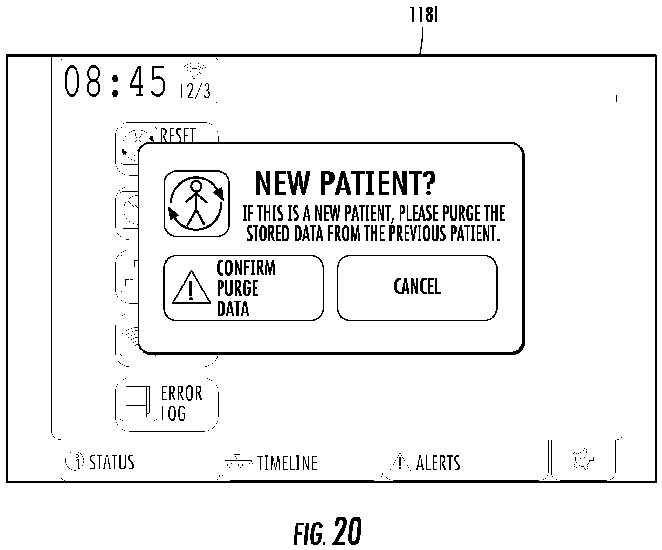

FIG. 20 shows an illustrative screen shot that may be displayed in response to a new patient being positioned on patient support 36.

### Local Alert Lights

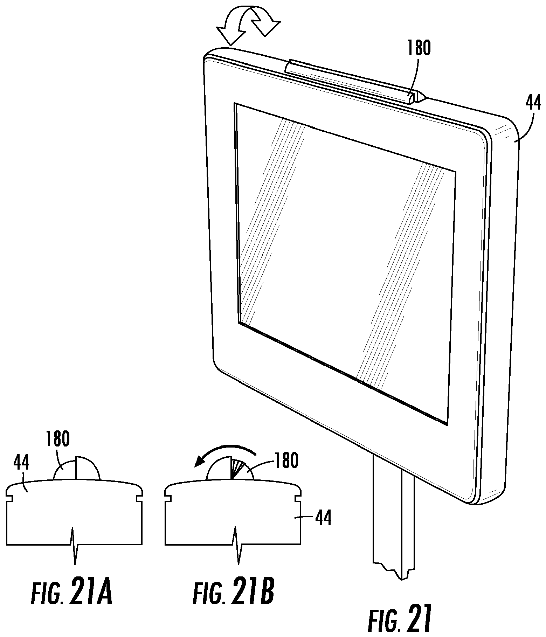

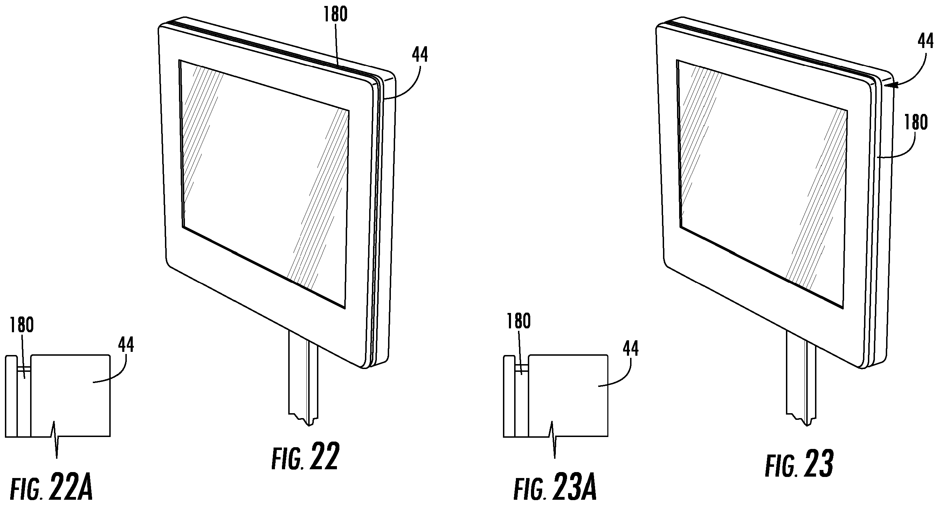

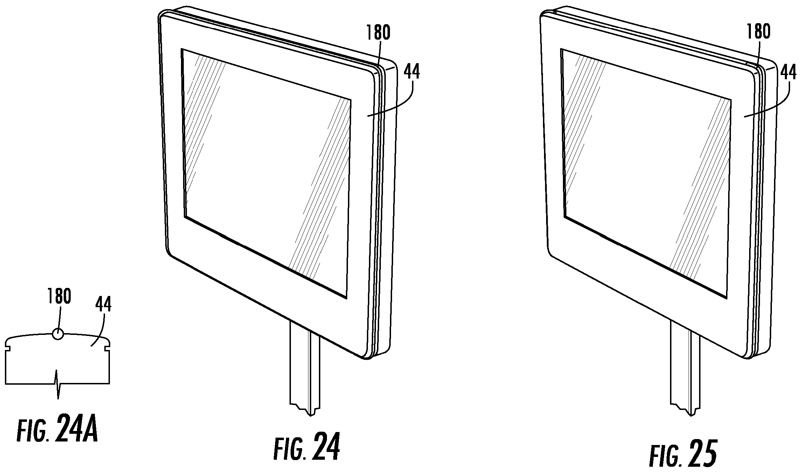

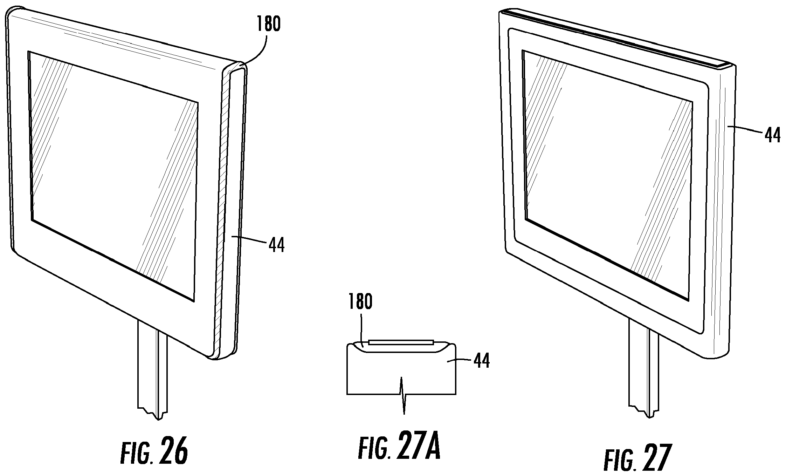

FIGS. 21-27 illustrate various manner in which one or more local alert lights 180 may be integrated into tablet 44. Such alert lights 180 may change color in response to any one or more of the conditions sensed by sensing system 20 changing from a non-alert state to a state in which an alert should be issued. Alert lights 180 may be positioned anywhere on tablet 44. In the embodiments shown, alert lights 180 are positioned around all or a portion of the perimeter of tablet 44. By positioning lights 180 in this manner, the alert light can be easily viewed by a caregiver who is not within the vicinity of tablet 44 or patient support 36. Thus, for example, a caregiver who is walking down a hospital hallway can easily view alert lights 180 from the hallway without having to enter the room to inspect system 20, or to read any of the contents displayed on display screen 48.

Alert lights 180 may also serve a dual role as a normal light. That is, alert lights 180 may also be illuminated when sensing system 20 is detecting conditions that do not justify an alert. To indicate that no alert conditions are currently being sensed, alert lights 180 may be illuminated in a color that is different from their color when they indicate an alert. As one example, lights 180 may be a shade of green when no alerts are currently detected by sensing system 20, and may change to an amber color when an alert is detected. Other color schemes may also be used. Lights 180 may also flash, or make other visual appearances, in response to an alert condition. By including the normal light function, a caregiver also does not need to approach tablet 44 or patient support 36 to view the status of sensing system 20. Instead, for example, the caregiver can see from a hallway-without entering a patient room-whether sensing system is operating properly without alerts (i.e. when the normal color is illuminated) or if an alert condition is present (the alert color is illuminated.)

FIGS. 21A, 21B, 22A, 23A, 24A, and 27A are partial cross sectional views of the edges of tablet 44 illustrating different manners in which lights 180 may be integrated into tablet 44. However configured, alert lights 180 may be constructed so that the light from the alerting lamp is easily viewable to a remotely positioned caregiver (e.g. from a hallway, or the like) regardless of which orientation the tablet 44 is in relative to pedestal 50. In some embodiments, tablet 44 may be rotatable on pedestal 50 in one or more directions. Lights 180 may be constructed such that, regardless of orientation, they are easily viewable to a remotely positioned caregiver.

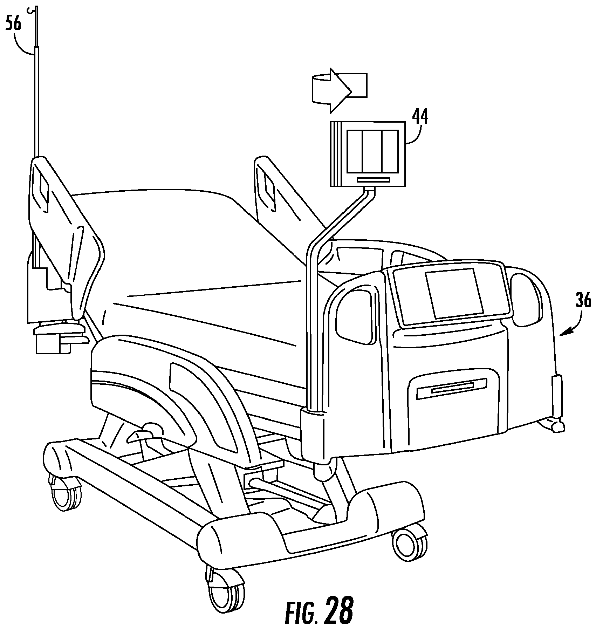

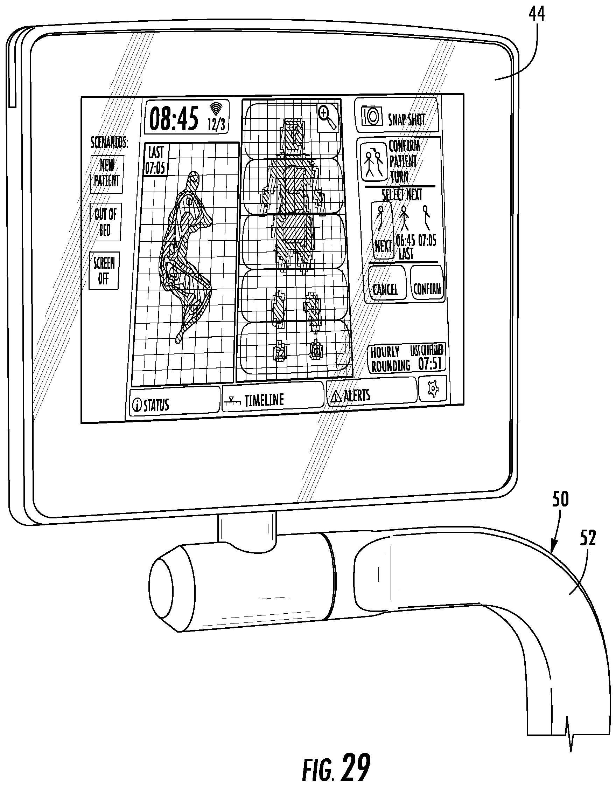

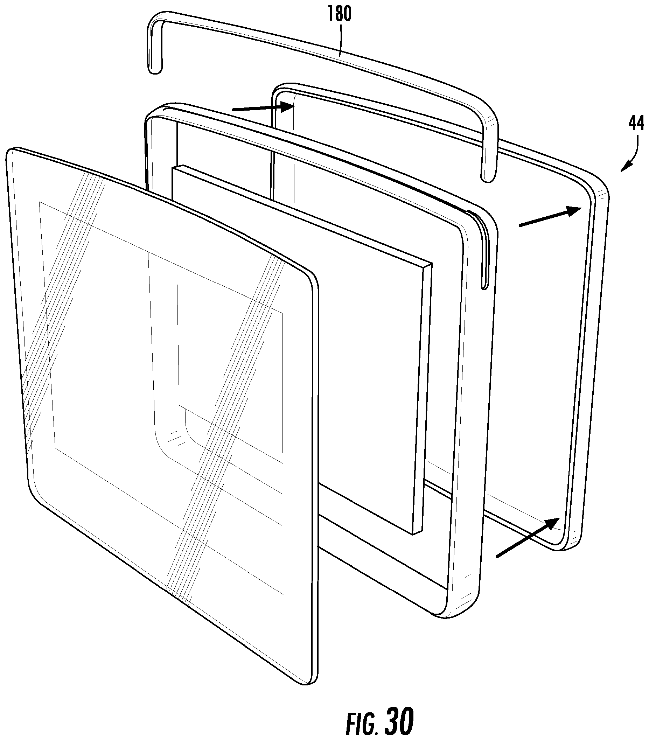

FIG. 28 illustrates an example of tablet 44 having been pivoted around a vertical axis such that the contents of display screen 48 are viewable to a caregiver standing adjacent the foot end of patient support 36. FIG. 29 illustrates a close up view of one example of tablet 44 and a portion of the pedestal 50 to which it is mounted. FIG. 30 shows an exploded view of the various components of one example of tablet 44. The reference to the "awareness light" refers to one example of an alert/normal light 180.

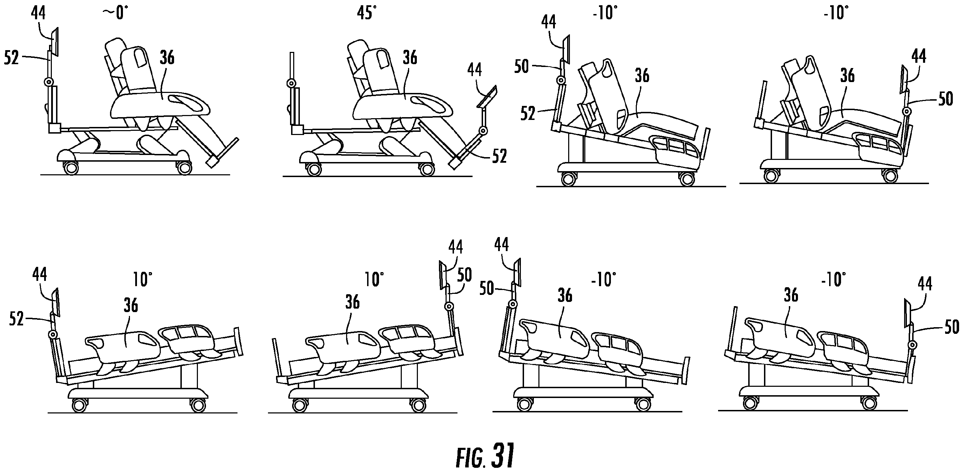

FIG. 31 shows several different orientations of patient support 36, as well as several different orientations and positions of pedestal 50 and tablet 44 when it is mounted to patient support 36. As can be seen, pedestal 50 can be mounted at either the foot end or the head end of patient support 36 and it does not interfere with the various movements and different orientations to which patient support 36 may be adjusted.

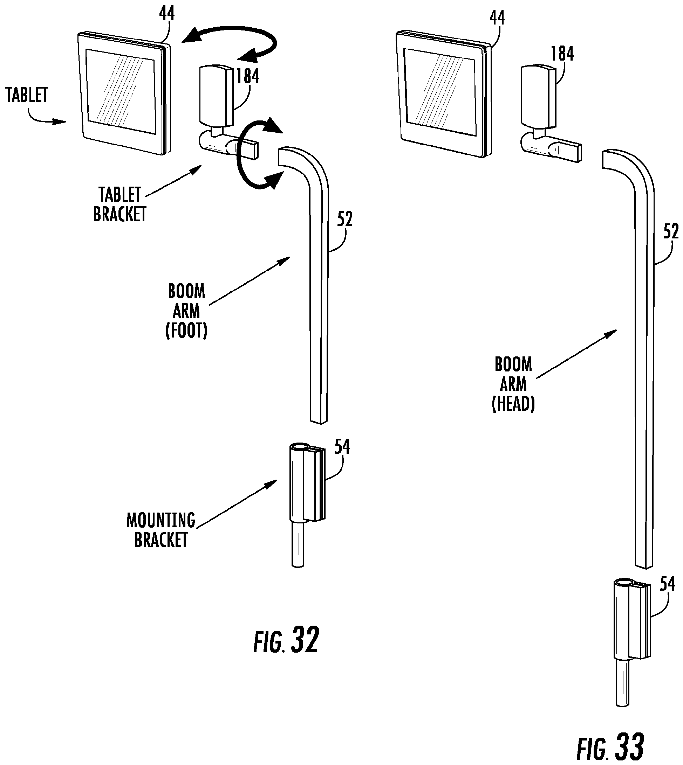

FIGS. 32 and 33 show two different versions of a pedestal 50 that may be used to support tablet 44. In each version, pedestal 50 includes boom arm 52, bracket 54, and a tablet bracket 184. Tablet bracket 184 may be constructed, such as shown in FIGS. 32 and 33, to be rotatable about both a horizontal and a vertical axis to thereby enable tablet 44 to be rotated about the same angles. This enables a caregiver to adjust the viewing angle of tablet 44 in both directions.

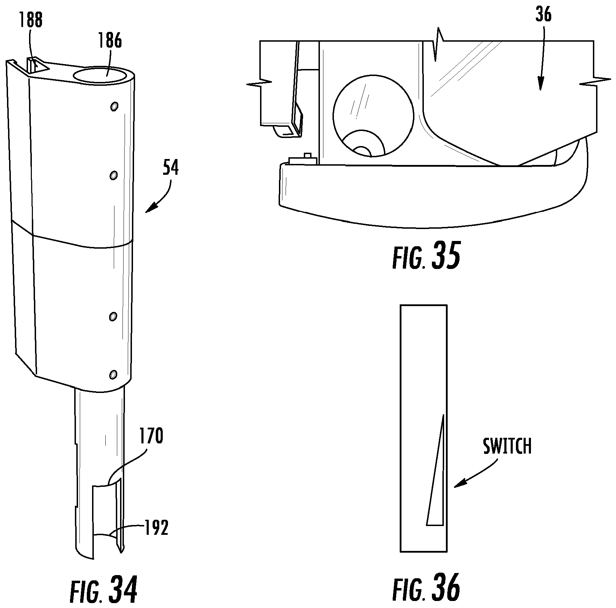

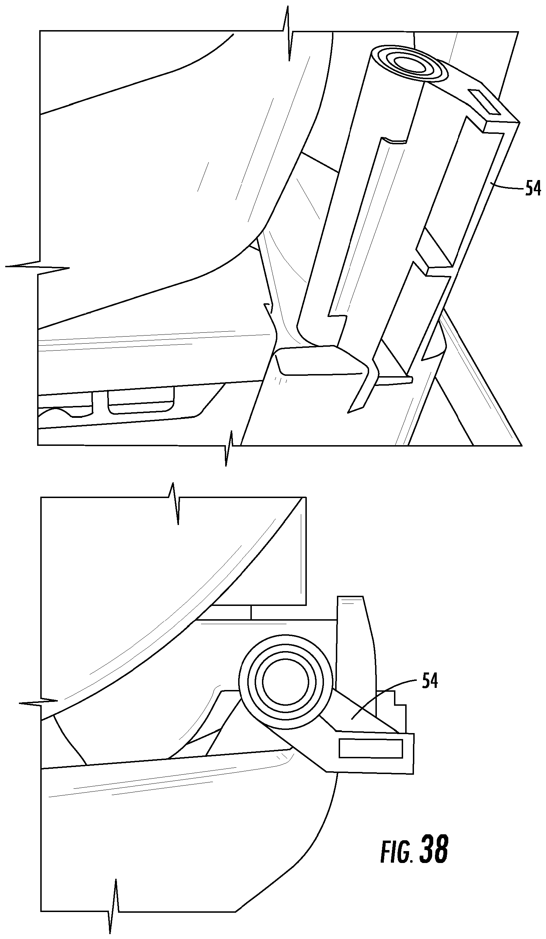

Further details of one embodiment of mounting bracket 54 are shown in FIG. 34. In that embodiment, mounting bracket 54 includes an IV pole slot 186, a boom slot 188, and an insert 190. Insert 190 is adapted to fit into a conventional IV pole hole or slot defined in patient support 36. FIG. 35 illustrates one such conventional IV pole hole defined in patient support 36. FIGS. 37-38 illustrate one embodiment of bracket 54 inserted into such an IV pole hole. In the embodiment of FIG. 34, boom slot 188 is adapted to releasably support boom arm 52. A caregiver can thus remove boom arm 52 from slot 188 by lifting boom arm upwardly. IV pole slot 186 is provided so that a conventional IV pole may be inserted thereinto. In this manner, the use of pedestal 50 does not prevent a caregiver from also mounting an IV pole to the same position on patient support 36. Slot 186 thus functions in the same manner as the IV pole hole of on patient support 36. As shown in FIGS. 34 and 36, bracket 54 may include a notched region 192 that cooperates with a switch (FIG. 36) when it is inserted into the IV pole hole.

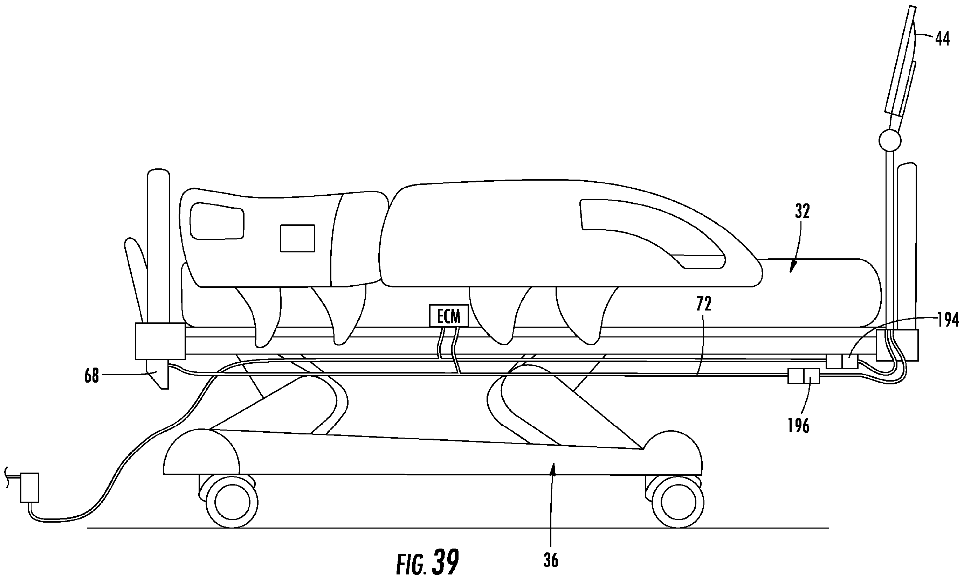

FIG. 39 illustrates one manner in which electrical cabling may be configured for supplying power and room location data to sensing system 20. More specifically, a power plug 194 is included that enables sensing system 20 to releasably connect to an electrical power supply, such as an electrical cable that is plugged into a conventional electrical wall outlet. A data plug 196 is also included that enables sensing system 20 to releasably connect to communication line 72, which transmits room location data from locator device 68 to system 20.

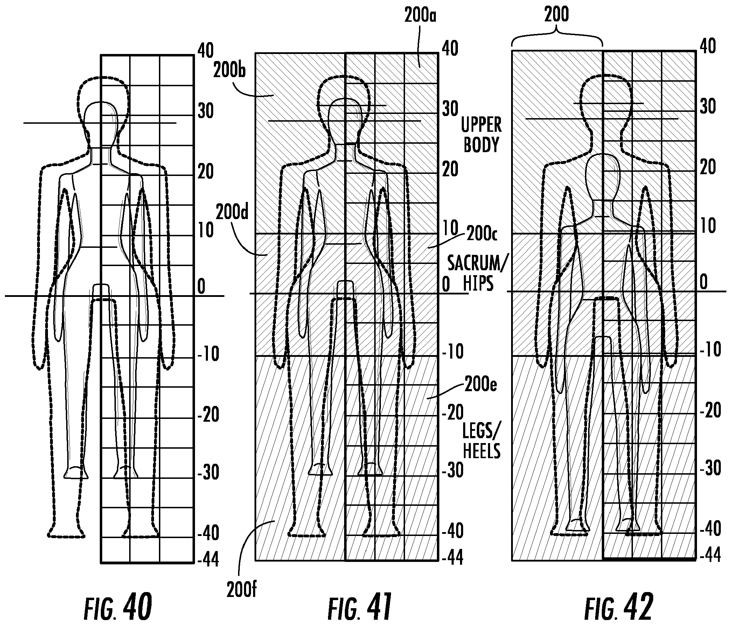

FIGS. 40-42 illustrate a plurality of zones 200 that may be defined with respect to sensor array 22. As shown, zones 200 are defined to correlate to specific regions of a patient's body. Thus, as shown, there may be an upper left body zone 200a, an upper right body zone 200b, a left sacrum zone 200c, a right sacrum zone 200d, a left leg zone 200e, and a right leg zone 200f. These zones are correlated to the sensor cells 110 within sensor array 22. Therefore, system 20 can correlate the output of a particular sensor cell 110 to one of these specific zones. It will be understood, of course, that fewer or greater numbers of zones may be defined than those illustrated in FIGS. 40-42.

The use of zones 200 allows sensing system 20 to monitor conditions in different manners based upon the specific zones. The different manners may be based upon inputs from a caregiver, or they may be carried out automatically without any input from the user, or a combination of the two. In some embodiments, the sensor cells corresponding to a particular zone are dynamic, rather than static. In such dynamic systems, the definition of a zone will change based upon the patient's location on sensor array 22. Thus, for example, if a patient slides down toward the foot end of patient support 36, sensing system 20 may shift the zones downward in the same direction so that the zone definitions maintain their correlation to the patient's body. In other words, if a patient slides down so far that his or her hips would otherwise be out of the sacrum zones 200c and d (in a static zone system), system 20 will automatically redefine the zones so that they match the patient's location on sensor array 22.

### Other Sensed Conditions

In addition to the conditions discussed above that may be sensed by sensing system 20, a number of other conditions may be sensed, either in addition to, or in lieu of, those conditions already discussed. For example, sensor array 22 may be used to detect a patient's heart rate and/or breathing rate. These may be detected via sensor cells 110. Sensor array 22 may also detect whether a patient needs a boost or not by monitoring the position of the patient on the support 36. If the patient moves downward over time, an alert may be issued indicating that the patient could use a boost while positioned on support 36. Additional sensors may be integrated into flexible mat 32 that detect moisture, due to such things as perspiration or incontinence. When so detected, an alert could be issued by system 20. Temperature sensors may also be included in flexible mat 32. If undesirable temperature conditions are detected (e.g. undue heat), an alert may be issued, and/or information regarding the alert may be forwarded to a surface controller that will cause the surface controller to take some action that mitigates the temperature condition.

Sensor array 22 may also be configured, in some embodiments, to determine the height of a patient lying on patient support 36. This may be done by determining which sensor cells 110 are detecting pressure from patient's head, and which are detecting pressure from the patient's feet, and then using the known physical distance between the cells to calculate the height. Adjustments may be made in the height calculation to account for the patient lying askew on the patient support, as well as to account for the fact that the very top of a patient's head and the very bottom of the patient's feet may not be exerting sufficient pressure to be detected by sensor cells 110. In such cases, sensing system 20 may infer from these endpoints from the other cells and/or a determination of the patient's position on mattress 38.

Once system 20 knows the height of the patient, it may be configured in some embodiments to automatically calculate the patient's body mass index (BMI). In order to do this, system 20 also needs to know the patient's weight. In some embodiments, sensor array 22 may be configured to measure this directly. In other embodiments, sensing system 20 may be configured to receive the weight information electronically from a scale system incorporated into patient support 36. In still other embodiments, a caregiver may input the patient's weight into system 20 via tablet 44, or through some other means. In still other embodiments, system 20 may retrieve the patient's weight automatically from an EMR.

In addition to computing conventional BMI, system 20 may be configured, in some embodiments, to calculate a regional BMI. A regional BMI may be calculated in the same manner as a conventional BMI, except adjusted to a specific region or portion of a patient's body. That is, the height (or length) and weight measurements of a particular portion of a patient's body may be calculated. For some embodiments, the regional BMI may correlate to specific zones, such as those shown in FIGS. 40-42, or to other zones.

In other embodiments, sensing system 20 may be configured such that regional bed exit alerts may be issued. Regional bed exit alerts may correspond to a patient moving off of a portion of sensor array 22, or moving in a manner that indicates potential exit from a region of sensor array 22, rather than exiting completely off of patient support 36.

System 20 may also be configured to detect if a patient is experiencing a seizure, or undue agitation, by monitoring the pressure readings of cells 110. If either condition is detected, an alert may be issued. Based upon the amount and/or kind of movement detected by sensor array 22 while a patient is sleeping, sensing system 20 may also be configured to generate a quality of sleep indicator. This indicator may then be communicated to the caregiver, or to an EMR, so that the caregivers are informed of the quality of the patient's sleep. The sleep indicator may be a numeric value, or any other suitable value or values.

System 20 may further be configured to suggest specific types of mattresses 38, or other surfaces, that would be advantageous for a particular patient. In doing this, system 20 may have access to a memory in which a hospital's inventory of mattress types, as well as their functionalities, are maintained. Based upon conditions sensed by sensor array 22, system 20 may issue a recommendation to a caregiver for a specific type of mattress 38 to be used with a particular patient. The recommendation may be based on, for example, detecting that a patient has bottomed out on a particular mattress, and therefore should be moved to a different kind of mattress that won't bottom out. As another example, a recommendation may be made for a different type of mattress because the different type of mattress has treatment capabilities, such as the ability to provide percussion therapy, or other types of treatments.

Sensing system 20 may also process the outputs from sensor cells 110 to determine if an entrapment hazard for the patient may exist. This may be accomplished by looking at the patient's location on support 36 (via sensor array 22) and determining if an undue amount of pressure is being exerted in a location that is near a side rail, or other edge region of support 36. This may indicate that the patient is in a location where a higher risk exists for getting entrapped between the mattress and a side rail of patient support 36, or between other objects. System 20 may then issue an alert so that a caregiver can come and help move the patient to a new location where the risk of entrapment is reduced.

Sensing system 20 may also be configured to detect foreign objects (i.e. non-patient objects) that are positioned on sensor array 22. This may be done by analyzing the outputs of sensor cells 110, determining the patient's location on the sensor array 22, and looking for detected pressure that constitutes either a location anomaly (based on the patient's location), or a pressure anomaly. Such anomalies may indicate the existence of a foreign object on the mattress 38. Such objects may be hard and may be undesirable, particularly if they are, or get positioned, under a patient. System 20 may issue an alert so that a caregiver will be summoned to remove the object from the mattress 38 if it is an undesired object.

In some embodiments, flexible mat 32 may include one or more proximity switches, or the like, that are integrated thereinto at locations that align with the side rails of patient support 36. The switches are adapted to detect if the side rail is in the up or down position. The status of the side rails (up or down) can then be determined by sensing system 20 and indicated to caregivers, either on tablet 44, or at a remote location, such as a nurse's station. If certain side rail states are desired, system 20 may be configured such that it will issue an alert if it detects that a side rail has moved to an undesired state.

Flexible mat 32 may also be configured to include within it one or more additional proximity sensors, or the like, that are integrated into the mat at locations that are adjacent the head board and the foot board of patient support 36. Such sensors are configured to detect the presence or absence of the head board and foot board. The status of the head board and foot board may then be indicated on tablet 44 and/or forwarded to a remote location, such as a nurse's station. The presence or absence of a foot board, for example, may be information that is desirably known because some patient's, such as diabetic patients, may push their feet against the footboard, to the detriment of blood circulation in their feet.

In still other embodiments, sensing system 20 may include one or more pulse oximeters integrated into it, whether incorporated into flexible mat 32, or otherwise. Pulse oximeters may provide indications about the quality of the patient's circulation, which are a factor in the patient's likelihood of developing pressure ulcers. The outputs from the pulse oximeter may be fed to one of the controllers within system 20 and used to automatically adjust the alert criteria for wound prevention alerts. That is, the worse the patient's circulation, the lower system 20 may set the threshold that initiates a wound prevention alert. In still other embodiments, the readings from a pulse oximeter that is not integrated into system 20 may be input into system 20 via user interface 26, or by other means, and system 20 may use this information to automatically adjust the alert criteria in an appropriate fashion.

In still other embodiments, flexible mat 32 may include an air cushioning ability for assisting in patient transfer. That is, flexible mat 32 may include a plurality of air holes defined on its underside and a connection to a pressurized air source. If it is desired to move a patient from one patient support to another surface, the pressurized air can be applied to flexible mat 32 such that the pressurized air escapes through the plurality of air holes. This escaping air creates an air cushion underneath the mat 32, which thereby reduces the frictional resistance to sliding mat 32-and the patient thereon-from one patient support 36 to another patient support, or other support surface. Details of an example of such air cushion transfer devices may be found in commonly assigned, copending U.S. patent application Ser. No. 12/554,431 filed Sept. 4, 2009 by applicants Schreiber et al. and entitled PATIENT TRANSFER DEVICE.

In some embodiments, a camera may be incorporated into system. Such a camera may be a still image camera, or a live-action camera. Such a camera may also be a digital camera that outputs digital images for analysis by any one of the controllers in system 20. The camera may be used for determining the patient position, or it may be used for taking pictures of a patient's wound, or of other aspects of the patient. Any pictures taken may be recorded for later viewing, either on a display of system 20, or at a remote location. Such a camera may be used to visually document the progress of healing, or the evolution of, a patient's wound. Such images may also be forwarded via system 20 to an EMR.

In some embodiments, a camera system that combines visual images with depth measurements, may be used in conjunction with sensing system 20 for determining patient position, and/or for determining other conditions or parameters. An example of such a system is disclosed in commonly assigned U.S. patent application serial number 13/242,022 filed Sept. 23, 2011, by applicants Richard Derenne et al. and entitled VIDEO MONITORING SYSTEM.

System 20 may also include any one or more of the following: a printer, a projector, and a scanner. The printer may allow a caregiver to print out data gathered by sensing system 20, including, but not limited to, pressure distribution profiles 112. The printer may be supported on the boom arm, integrated into tablet 44, or otherwise positioned in a convenient location for a caregiver to have documents printed.

The projector may project images onto a nearby screen for viewing. The projector may also project light with various colors onto the patient in such a manner so that the colors are projected onto the body in a manner that matches the pressure being experienced by the patient. In other words, the projector may be configured to project a pattern of light that matches the pressure distribution profile 112 onto the patient. The colors of profile 112 will then be projected onto the patient's physical body at the corresponding location. In this manner, a caregiver can easily see where on the patient's body he or she is experiencing high pressure, low pressure, etc.

In another embodiment, the projector may be configured to project one or more images of a control panel onto flexible mat 32 at a predefined or known location. When a user physically presses that region of mat 32, sensor cells 110 will detect this pressing, and depending upon the location of the physical pressing and the image projected onto that location, sensing system 20 will react in the appropriate manner. In other words, the projector can be used to project a virtual control panel onto flexible mat 32 so that the caregiver can control aspects of system 20 simply by pressing the appropriate location on mat 32.

If a scanner is included in sensing system 20, the scanner may be used to scan identification cards, badges, or the like, which may be carried by caregivers. Once scanned, system 20 will determine the identity of the caregiver and will then record whatever therapies, changes, or other events that transpire under the supervision of that particular caregiver. For example, if the patient is being turned, system 20 will be able to automatically record the identity of the caregiver who performed the patient turn (as well as the time of the turn, the direction of the turn, and any other useful information).

In other embodiments, sensing system 20 may include one or more sensor arrays 22 that are integrated into objects other than flexible mat 32. In some of these embodiments, such sensor arrays may be in addition to the one or more that are part of mat 32. In other embodiments, such sensor arrays may be the only sensor arrays in system 20. Such sensor arrays may be incorporated into other mats, or still other devices. For example, additional sensor arrays may be positioned in one or more pillows so that pressure readings can be taken from the pillows, or other objects. These additional sensor readings may then be forwarded to one of the controllers in system 20 so that all of the pressures being experienced by a patient may be measured and communicated to a caregiver. In some embodiments, one or more sensor arrays may be incorporated into, or attached to, the foot board and/or head board of patient support 36 so that the pressure exerted by any of the patient's body parts thereagainst can be monitored.

In other embodiments, sensor arrays 22 may be incorporated into fabric, or other material, that is worn by a patient, or adhered to the patient. The readings from these sensor arrays may then be forwarded to one of the controllers of sensing system 22 for processing. Such wearable sensors may include adhesives positioned along a portion of their surface area for releasably attaching the sensor sheet to a patient. In still other embodiments, regional patches having sensor arrays 22 may be adhered to areas of mattress 38, or to other surfaces on which the patient may lie, or to specific body areas on the patient. In still other embodiments, sensor arrays 22 may be incorporated into bandages for wounds. This type of sensor would provide data that would indicate whether a patient's wound was experiencing undue pressure, as well as other data. In still other embodiments, sensor arrays 22 may be incorporated into mats, or the like, that are positioned on the floor so that a patient can walk on them. The sensed pressures may be used for gait assessment, or other clinical purposes.

In still other embodiments, one or more additional sensors may be incorporated into other patient support surfaces on which a patient may be positioned. Such patient support surfaces may include recliners, chairs, OR tables, or other surfaces. The pressure readings taken from these sensor arrays may be forwarded to one of the controllers in sensing system 20 where it can be taken into account when monitoring the patient while in patient support 36. For example, if a patient is on a first support surface and is experiencing a lot of pressure for a certain amount of time while positioned on, say, an OR table, this information may be communicated to a controller in sensing system 20. The controller may then use this information while monitoring the patient when he or she is positioned on patient support 36. For example, the system may use this information to adjust the alerting criteria. In other words, if pressure conditions existed while on the OR table, these would be communicated to a controller of system 20 so that an alert might be issued sooner due to the cumulative pressure readings experienced by the patient both on the OR table and on patient support 36. As an example, if a patient's left leg experienced high pressure on the OR table, when he or she was transferred to patient support 36, system 20 would use this information to adjust the alert criteria associated with the patient's leg, thereby issuing a pressure alert sooner that it might otherwise have done had the patient not experienced high pressure in his or her leg while on the OR table.

In any of the embodiments discussed above, the flexible mat 32 and/or controller 24 associated with one or more particular sensing arrays 22 may include a unique identifier that distinguishes a particular mat 32 and/or controller 24 from other mats 32 and/or controllers 24. This mat or controller ID may then be transmitted to the hospital network with the data detected by sensor array 22 so that a server, or other device on the network, could determine which specific mat 32 and/or controller 24 was transmitting the data in a healthcare institution. The server or other computer device could maintain a correlation between the ID and one or more additional items, or could be programmed to determine a correlation between the ID and one or more additional items. The additional items could include a patient ID, a caregiver ID, a bed ID, a mattress ID, and/or other information. Thus, for example, if multiple mats 32 were positioned in a room and were to be associated with one specific patient, the unique IDs of those mats could be stored and correlated with a single patient ID so that all data transmitted from those mats were associated with the corresponding patient.

Still further, in some embodiments, sensing mat 32 may communicate with a mattress positioned on a bed, or a cushion on a chair, or with another cushioning structure, via a Universal Serial Bus (USB) cable. The cable may have one end that plugs into the mat 32 and an opposite end that plugs into the bed, cushion, or other structure. In some embodiments, the bed may include a USB port for coupling the cable wherein the USB port allows communication with a mattress positioned thereon.

In still other embodiments, sensing mat 32 may communicate with patient support apparatus 36 and provide signals that are used to control the movement of one or more portions of patient support apparatus. For example, forces exerted by a caregiver on one or more specific sections of flexible mat 32 may be used to raise or lower the height of patient support apparatus 36, or to change the orientation of one or more sections of patient support apparatus 36. The use of sensing mat 32 would provide additional manners for a user to control patient support apparatus 36 beyond the conventional apparatus controls that are typically found in siderail control panels and/or footboard control panels. The force sensors in flexible mat 32 that could be used for controlling patient support 36 movement could be located along the sides of mat 32, or near one or more of the corners of mat 32. In some embodiments, the force sensors of array 22 could be used as the force sensors referenced by the number 90 in commonly assigned, U.S. patent application Ser. No. 61/599,099, filed Feb. 15, 2012, entitled PATIENT SUPPORT APPARATUS AND CONTROLS THEREFOR, by applicants Richard Derenne.

It will be understood by those skilled in the art that any of the features described in any of the various embodiments discussed herein may be combined with any one or more of the other features described in the other embodiments discussed herein. It will also be understood that, while the present invention has been described herein in terms of the several embodiments illustrated in the attached drawings, it will be understood by those skilled in the art that the present invention can be modified to include any and all variations that are within the scope of the following claims.

## Claims

1. A sensing system (20) for a patient support (36) comprising:
a sensor array (22) integrated into a flexible mat (32), said mat (32) adapted to be placed over a patient support surface positioned on the patient support (36), said sensor array (22) including a plurality of sensors (110) that are each adapted to detect perpendicular force exerted by a patient onto a respective sensor (110) in the sensor array (22);
a controller (28) in communication with said sensor array (22) and adapted to process outputs from said sensor array (22) to determine a pressure distribution profile (112) of a patient positioned on the patient support (36); and
a display screen (48) supported on the patient support (36), said display screen (48) adapted to display the pressure distribution profile (112); wherein said controller (28) is adapted to determine from said sensor array (22) if an undesirable shear condition exists, wherein said undesirable shear condition is based upon said sensor array (22) detecting movement along said sensor array (22) of an area of pressure exerted by the patient against the sensor array (22), said undesirable condition being detected only if said area of pressure exceeds a predetermined size, only if a magnitude of pressure within said area of pressure exceeds a predetermined threshold, and only if a rate of the movement exceeds a predetermined threshold.

2. The system (20) of claim 1 wherein said controller (28) is further adapted to map the plurality of sensors (110) in said sensor array (22) to at least one body part of a patient on the patient support (36).

3. The system (20) of claim 1 further including a user interface (26) supported on the patient support (36) and associated with the display screen (48), said user interface (26) in communication with said controller (28), said user interface (26) adapted to allow a caregiver to select which of said plurality of sensors (110) in the sensor array (22) are used in a calculation performed by said controller (28).

4. The system (20) of claim 1 wherein said controller (28) is adapted to determine from said sensor array (22) at least one of the following: whether a patient has turned or not, if a patient is sitting up on the patient support (36), an angular orientation of a pivotable portion of the patient support surface, if an undesirable condition exists with respect to shear pressure exerted between the patient and the patient support surface, if a patient has bottomed out on a surface positioned underneath the patient, or if a patient has exited the patient support (36).

5. The system (20) of claim 1 wherein said controller (28) is adapted to issue an alert when at least one of the following conditions occurs: a patient may need boosting on the patient support (36), a patient may need to be turned, a patient may be experiencing excessive shear stress, a patient may be experiencing excessive compressive stress, a patient may have changed from a supine to a sitting position, a patient may have exited the patient support (36), and an angle of a head section of said patient support (36) may have decreased below a threshold.

6. The system (20) of claim 1 wherein said sensor array (22) and said controller (28) are adapted to determine at least one of the following: a patient's height; a patient's body mass index; a patient's heart rate, a patient's breathing rate, a patient's temperature, a patient's regional mass index, a patient's weight, a patient's quality of sleep, or a patient's level of agitation.

7. The system (20) of claim 1 wherein said controller (28) determines a location of a patient's torso on said sensor array (22) and said controller (28) uses said location of the patient's torso in determining a heart rate of the patient.

8. The system (20) of claim 1 wherein said controller (28) determines a location of a patient's torso on said sensor array (22) and said controller (28) uses said location of the patient's torso in determining a respiration rate of the patient.

9. The system (20) of claim 1 further including a temperature sensor integrated into said sensor array (22), said controller (28) adapted to use temperature values from said temperature sensor to adjust force values detected by said sensor array (22) in order to compensate for temperature dependent errors in said force values.

10. The system (20) of claim 1 wherein said user interface (26) enables a caregiver to select an area on said sensor array (22) that, in the presence of a threshold amount of patient movement detected on said selected area, will cause the user interface (26) to provide an indication discernible to the caregiver.

11. The system (20) of claim 1 wherein said controller (28) stores data sensed by the sensor array (22) in a memory, analyses the stored data to determine at least one patient tendency, and provides a recommendation to the caregiver about an action the caregiver can take to better care for the patient in light of the patient tendency, said patient tendency including at least one of the following: sleeping on a particular side of the patient support (36), using more than one pillow, or exiting the patient support (36) from a particular location.

12. The system (20) of claim 1 wherein said controller (28) is adapted to use the outputs of the sensor array (22) to determine a patient's location on the sensor array (22), and said controller (28) is further adapted to use the patient's location to initially define a plurality of zones (200) on said sensor array (22), said controller (28) dynamically adjusting the boundaries of said plurality of zones (200) if the patient changes location on the patient support surface, and said user interface (26) enabling a caregiver to select at least one of said zones (200) to base an alert condition on.

13. A method of upgrading a patient support (36) that does not communicate data to a remote location to an upgraded patient support (36) that does communicate data to a remote location, said method comprising:
providing a flexible mat (32) having a sensor array (22) integrated therein, said sensor array (22) including a plurality of sensors (110) that are each adapted to detect perpendicular force exerted by a patient onto a respective sensor (110) in the sensor array (22);
placing said mat (32) over a patient support surface positioned on the patient support (36);
providing a controller (28) separate from the patient support (36) to process outputs from said sensor array (22) to determine a pressure distribution profile (112) of the patient;
displaying the pressure distribution profile (112) of the patient on a display screen (48) supported on the patient support (36);
communicating the pressure distribution profile (112) of the patient off the bed to a remote location;
determining from said sensor array (22) if an undesirable shear condition exists by detecting movement along said sensor array (22) of an area of pressure exerted by the patient against the sensor array (22), said undesirable condition being detected only if said area of pressure exceeds a predetermined size, only if a magnitude of pressure within said area of pressure exceeds a predetermined threshold, and only if a rate of the movement exceeds a predetermined threshold;
issuing an alert if said undesirable condition is detected.

14. The method of claim 13 further including:
using said sensor array (22) and the controller (28) to detect when a patient has exited the patient support (36); and
communicating an alert signal from the patient support (36) to the remote location indicating that the patient has exited the patient support (36).

## Patentansprüche

1. Sensorsystem (20) für eine Patientenliege (36), umfassend:
ein in einer flexiblen Matte (32) integriertes Sensor-Array (22), wobei die Matte (32) dafür geeignet ist, über einer Patientenliegefläche platziert zu werden, die auf der Patientenliege (36) positioniert ist, wobei das Sensor-Array (22) eine Vielzahl von Sensoren (110) beinhaltet, die jeweils dazu geeignet sind, eine senkrechte Kraft zu erfassen, die von einem Patienten auf einen jeweiligen Sensor (110) in dem Sensor-Array (22) ausgeübt wird;
eine Steuereinrichtung (28) in Kommunikation mit dem Sensor-Array (22) und dafür geeignet, Ausgaben von dem Sensor-Array (22) zu verarbeiten, um ein Druckverteilungsprofil (112) eines auf der Patientenliege (36) positionierten Patienten zu bestimmen; und
einen Anzeigebildschirm (48), der auf der Patientenliege (36) gestützt ist, wobei der Anzeigebildschirm (48) dafür geeignet ist, das Druckverteilungsprofil (112) anzuzeigen;
wobei die Steuereinheit (28) dafür geeignet ist, aus dem Sensor-Array (22) zu bestimmen, ob ein unerwünschter Scherzustand vorliegt, wobei der unerwünschte Scherzustand darauf basiert, dass das Sensor-Array (22) eine Bewegung entlang des Sensor-Arrays (22) eines Bereichs eines Drucks erfasst, der von dem Patienten auf das Sensor-Array (22) ausübt wird, wobei der unerwünschte Zustand nur erfasst wird, wenn der Druckbereich eine vorbestimmte Größe überschreitet, nur wenn eine Größe des Drucks innerhalb des Druckbereichs einen vorbestimmten Schwellenwert überschreitet und nur wenn eine Rate der Bewegung einen vorbestimmten Schwellenwert überschreitet.

2. System (20) von Anspruch 1, wobei die Steuereinheit (28) ferner dazu geeignet ist, die Vielzahl von Sensoren (110) in dem Sensor-Array (22) auf mindestens einen Körperteil eines Patienten auf der Patientenliege (36) abzubilden.

3. System (20) nach Anspruch 1, ferner beinhaltend eine Benutzerschnittstelle (26), die auf der Patientenliege (36) gestützt und dem Anzeigebildschirm (48) zugeordnet ist, wobei die Benutzerschnittstelle (26) in Kommunikation mit der Steuereinheit (28) steht, wobei die Benutzerschnittstelle (26) dazu geeignet ist, es einer Pflegekraft zu ermöglichen, auszuwählen, welche der Vielzahl von Sensoren (110) in dem Sensor-Array (22) in einer von der Steuereinheit (28) durchgeführten Berechnung verwendet werden.

4. System (20) nach Anspruch 1, wobei die Steuereinheit (28) dazu geeignet ist, von dem Sensor-Array (22) mindestens eines von Folgendem zu bestimmen: ob sich ein Patient gedreht hat oder nicht, ob ein Patient auf der Patientenliege (36) aufrecht sitzt, eine Winkelausrichtung eines schwenkbaren Abschnitts der Patientenliegefläche, ob ein unerwünschter Zustand in Bezug auf den zwischen dem Patienten und der Patientenliegefläche ausgeübten Scherdruck vorliegt, ob ein Patient auf einer unter dem Patienten positionierten Oberfläche aufsetzt oder ob ein Patient die Patientenliege (36) verlassen hat.

5. System (20) nach Anspruch 1, wobei die Steuereinheit (28) dazu geeignet ist, eine Warnung auszugeben, wenn mindestens eine der folgenden Bedingungen eintritt: ein Patient müsste eventuell auf der Patientenliege (36) angehoben werden, ein Patient müsste eventuell gedreht werden, ein Patient könnte eventuell einer übermäßigen Scherbeanspruchung ausgesetzt sein, ein Patient könnte eventuell einer übermäßigen Druckbelastung ausgesetzt sein, ein Patient könnte eventuell von einer Rückenlage in eine sitzende Position gewechselt haben, ein Patient könnte eventuell die Patientenliege (36) verlassen haben und ein Winkel eines Kopfabschnitts der Patientenliege (36) könnte eventuell unter einen Schwellenwert gesunken sein.

6. System (20) nach Anspruch 1, wobei das Sensor-Array (22) und die Steuereinheit (28) dazu geeignet sind, mindestens eines von Folgendem zu bestimmen: eine Größe des Patienten; einen Body-Mass-Index des Patienten; eine Herzfrequenz des Patienten, eine Atemfrequenz des Patienten, eine Temperatur des Patienten, einen regionalen Masse-Index des Patienten, ein Gewicht des Patienten, eine Schlafqualität des Patienten oder ein Erregungsniveau des Patienten.

7. System (20) nach Anspruch 1, wobei die Steuereinheit (28) eine Lage eines Oberkörpers des Patienten auf dem Sensor-Array (22) bestimmt, und die Steuereinheit (28) die Lage des Oberkörpers des Patienten beim Bestimmen einer Herzfrequenz des Patienten verwendet.

8. System (20) nach Anspruch 1, wobei die Steuereinheit (28) eine Lage eines Oberkörpers des Patienten auf dem Sensor-Array (22) bestimmt und die Steuereinheit (28) die Lage des Oberkörpers des Patienten beim Bestimmen einer Atmungsrate des Patienten verwendet.

9. System (20) nach Anspruch 1, ferner beinhaltend einen in das Sensor-Array (22) integrierten Temperatursensor, wobei die Steuereinheit (28) dazu geeignet ist, Temperaturwerte von dem Temperatursensor zu verwenden, um von dem Sensor-Array (22) erfasste Kraftwerte einzustellen, um temperaturabhängige Fehler bei den Kraftwerten auszugleichen.

10. System (20) nach Anspruch 1, wobei es die Benutzerschnittstelle (26) einer Pflegekraft ermöglicht, einen Bereich auf dem Sensor-Array (22) auszuwählen, der beim Vorliegen einer Schwellenmenge an Patientenbewegung, die auf dem ausgewählten Bereich erfasst wurde, die Benutzerschnittstelle (26) veranlasst, eine Angabe bereitzustellen, die von der Pflegekraft erkannt werden kann.

11. System (20) nach Anspruch 1, wobei die Steuereinheit (28) Daten in einem Speicher speichert, die von dem Sensor-Array (22) erfasst wurden, die gespeicherten Daten analysiert, um mindestens eine Patiententendenz zu bestimmen und der Pflegekraft eine Empfehlung hinsichtlich einer Maßnahme bereitstellt, welche die Pflegekraft ergreifen kann, um den Patienten im Lichte der Patiententendenz besser zu versorgen, wobei die Patiententendenz mindestens eines von Folgendem beinhaltet: Schlafen auf einer bestimmten Seite der Patientenliege (36), Verwenden von mehr als einem Kissen oder Verlassen der Patientenliege (36) von einer bestimmten Lage aus.

12. System (20) nach Anspruch 1, wobei die Steuereinheit (28) dazu geeignet ist, die Ausgänge des Sensor-Arrays (22) zu verwenden, um eine Lage des Patienten auf dem Sensor-Array (22) zu bestimmen, und die Steuereinheit (28) ferner dazu geeignet ist, die Lage des Patienten zu verwenden, um anfänglich eine Vielzahl von Zonen (200) auf dem Sensor-Array (22) zu definieren, wobei die Steuereinheit (28) die Grenzen der Vielzahl von Zonen (200) dynamisch anpasst, wenn der Patient die Lage auf der Patientenliegefläche ändert und die Benutzerschnittstelle (26) es einer Pflegekraft ermöglicht, mindestens eine der Zonen (200) auszuwählen, auf denen eine Alarmbedingung basiert.

13. Verfahren zum Aufrüsten einer Patientenliege (36), die keine Daten an einen entfernten Ort übermittelt, zu einer aufgerüsteten Patientenliege (36), die Daten an einen entfernten Ort übermittelt, wobei das Verfahren umfasst:
Bereitstellen einer flexiblen Matte (32) mit einem darin integrierten Sensor-Array (22), wobei das Sensor-Array (22) eine Vielzahl von Sensoren (110) beinhaltet, die jeweils dazu geeignet sind, eine senkrechte Kraft zu erfassen, die von einem Patienten auf einen entsprechenden Sensor (110) in dem Sensor-Array (22) ausgeübt wird;
Platzieren der Matte (32) über einer auf der Patientenliege (36) positionierten Patientenliegefläche;
Bereitstellen einer von der Patientenliege (36) getrennten Steuereinheit (28) zum Verarbeiten von Ausgaben von dem Sensor-Array (22), um ein Druckverteilungsprofil (112) des Patienten zu bestimmen;
Anzeigen des Druckverteilungsprofils (112) des Patienten auf einem Anzeigebildschirm (48), der auf der Patientenliege (36) gestützt ist;
Übermitteln des Druckverteilungsprofils (112) des Patienten vom Bett an einen entfernten Ort;
Bestimmen aus dem Sensor-Array (22), ob ein unerwünschter Scherzustand vorliegt, indem eine Bewegung entlang des Sensor-Arrays (22) eines Bereiches eines Drucks, der von dem Patienten auf das Sensor-Array (22) ausgeübt wird, erfasst wird, wobei der unerwünschte Zustand nur erfasst wird, wenn der Druckbereich eine vorbestimmte Größe überschreitet, nur wenn eine Größe des Drucks innerhalb des Druckbereichs einen vorbestimmten Schwellenwert überschreitet und nur wenn eine Rate der Bewegung einen vorbestimmten Schwellenwert überschreitet,
Ausgeben eines Alarms, wenn der unerwünschte Zustand erfasst wird.

14. Verfahren nach Anspruch 13, ferner beinhaltend:
Verwenden des Sensor-Arrays (22) und der Steuereinheit (28), um zu erfassen, wann ein Patient die Patientenliege (36) verlassen hat; und
Übermitteln eines Alarmsignals von der Patientenliege (36) an den entfernten Ort, das anzeigt, dass der Patient die Patientenliege (36) verlassen hat.

## Revendications

1. Système de détection (20) pour un support de patient (36) comprenant :
un réseau de capteurs (22) intégré dans un tapis flexible (32), ledit tapis (32) étant conçu pour être placé sur une surface de support de patient positionnée sur le support de patient (36), ledit réseau de capteurs (22) comportant une pluralité de capteurs (110) qui sont chacun conçus pour détecter une force perpendiculaire exercée par un patient sur un capteur respectif (110) dans le réseau de capteurs (22) ;
un dispositif de commande (28) en communication avec ledit réseau de capteurs (22) et conçu pour traiter des sorties dudit réseau de capteurs (22) afin de déterminer un profil de distribution de pression (112) d'un patient positionné sur le support de patient (36) ; et
un écran d'affichage (48) supporté sur le support de patient (36), ledit écran d'affichage (48) étant conçu pour afficher le profil de distribution de pression (112) ;
dans lequel ledit dispositif de commande (28) est conçu pour déterminer à partir dudit réseau de capteurs (22) si une condition de cisaillement indésirable existe, dans lequel ladite condition de cisaillement indésirable est basée sur ledit réseau de capteurs (22) détectant un mouvement le long dudit réseau de capteurs (22) d'une zone de pression exercée par le patient contre le réseau de capteurs (22), ladite condition indésirable étant détectée uniquement si ladite zone de pression dépasse une taille prédéterminée, uniquement si une grandeur de pression à l'intérieur de ladite zone de pression dépasse un seuil prédéterminé, et uniquement si un taux du mouvement dépasse un seuil prédéterminé.

2. Système (20) selon la revendication 1, dans lequel ledit dispositif de commande (28) est en outre conçu pour mettre en correspondance la pluralité de capteurs (110) dans ledit réseau de capteurs (22) avec au moins une partie du corps d'un patient sur le support de patient (36).

3. Système (20) selon la revendication 1, comportant en outre une interface utilisateur (26) supportée sur le support de patient (36) et associée à l'écran d'affichage (48), ladite interface utilisateur (26) étant en communication avec ledit dispositif de commande (28), ladite interface utilisateur (26) étant conçue pour permettre à un soignant de sélectionner lequel de ladite pluralité de capteurs (110) dans le réseau de capteurs (22) est utilisé dans un calcul effectué par ledit dispositif de commande (28).

4. Système (20) selon la revendication 1, dans lequel ledit dispositif de commande (28) est conçu pour déterminer à partir dudit réseau de capteurs (22) au moins l'un des éléments suivants :
si un patient s'est retourné ou non, si un patient est assis sur le support de patient (36), une orientation angulaire d'une partie pivotante de la surface de support de patient, si une condition indésirable existe en ce qui concerne la pression de cisaillement exercée entre le patient et la surface de support de patient, si un patient a atteint le fond sur une surface positionnée sous le patient, ou si un patient est sorti du support de patient (36).

5. Système (20) selon la revendication 1, dans lequel ledit dispositif de commande (28) est conçu pour émettre une alerte lorsqu'au moins l'une des conditions suivantes se produit : un patient peut avoir besoin de soutien sur le support de patient (36), un patient peut avoir besoin d'être tourné, un patient peut subir une contrainte de cisaillement excessive, un patient peut subir une contrainte de compression excessive, un patient peut être passé d'une position couchée à assise, un patient peut avoir quitté le support de patient (36) et un angle d'une section de tête dudit support de patient (36) peut avoir diminué en dessous d'un seuil.

6. Système (20) selon la revendication 1, dans lequel ledit réseau de capteurs (22) et ledit dispositif de commande (28) sont conçus pour déterminer au moins l'un des éléments suivants : la taille d'un patient ; l'indice de masse corporelle d'un patient ; la fréquence cardiaque d'un patient, la fréquence respiratoire d'un patient, la température d'un patient, l'indice de masse régional d'un patient, le poids d'un patient, la qualité de sommeil d'un patient ou le niveau d'agitation d'un patient.

7. Système (20) selon la revendication 1, dans lequel ledit dispositif de commande (28) détermine un emplacement du torse d'un patient sur ledit réseau de capteurs (22) et ledit dispositif de commande (28) utilise ledit emplacement du torse du patient pour déterminer une fréquence cardiaque du patient.

8. Système (20) selon la revendication 1, dans lequel ledit dispositif de commande (28) détermine un emplacement du torse d'un patient sur ledit réseau de capteurs (22) et ledit dispositif de commande (28) utilise ledit emplacement du torse du patient pour déterminer une fréquence respiratoire du patient.

9. Système (20) selon la revendication 1, comportant en outre un capteur de température intégré dans ledit réseau de capteurs (22), ledit dispositif de commande (28) étant conçu pour utiliser des valeurs de température dudit capteur de température pour ajuster des valeurs de force détectées par ledit réseau de capteurs (22) afin de compenser des erreurs dépendant de la température dans lesdites valeurs de force.

10. Système (20) selon la revendication 1, dans lequel ladite interface utilisateur (26) permet à un soignant de sélectionner une zone sur ledit réseau de capteurs (22) qui, en présence d'une quantité seuil de mouvement de patient détecté sur ladite zone sélectionnée, amènera l'interface utilisateur (26) à fournir une indication perceptible au soignant.

11. Système (20) selon la revendication 1, dans lequel ledit dispositif de commande (28) stocke des données détectées par le réseau de capteurs (22) dans une mémoire, analyse les données stockées pour déterminer au moins une tendance du patient, et fournit une recommandation au soignant concernant une action que le soignant peut prendre pour mieux soigner le patient à la lumière de la tendance du patient, ladite tendance du patient comportant au moins l'un des éléments suivants : dormir sur un côté particulier du support de patient (36), utiliser plusieurs oreillers, ou sortir du support de patient (36) à partir d'un emplacement particulier.

12. Système (20) selon la revendication 1, dans lequel ledit dispositif de commande (28) est conçu pour utiliser les sorties du réseau de capteurs (22) afin de déterminer l'emplacement d'un patient sur le réseau de capteurs (22), et ledit dispositif de commande (28) est en outre conçu pour utiliser l'emplacement du patient afin de définir initialement une pluralité de zones (200) sur ledit réseau de capteurs (22), ledit dispositif de commande (28) ajustant dynamiquement les limites de ladite pluralité de zones (200) si le patient change d'emplacement sur la surface de support de patient, et ladite interface utilisateur (26) permettant à un soignant de sélectionner au moins l'une desdites zones (200) sur laquelle baser une condition d'alerte.

13. Procédé de mise à niveau d'un support de patient (36) qui ne communique pas de données à un emplacement distant vers un support de patient mis à niveau (36) qui communique des données à un emplacement distant, ledit procédé comprenant :
la fourniture d'un tapis flexible (32), sur lequel est intégré un réseau de capteurs (22), ledit réseau de capteurs (22) comportant une pluralité de capteurs (110) qui sont chacun conçus pour détecter une force perpendiculaire exercée par un patient sur un capteur respectif (110) dans le réseau de capteurs (22) ;
le placement dudit tapis (32) sur une surface de support de patient positionnée sur le support de patient (36) ;
la fourniture d'un dispositif de commande (28) séparé du support de patient (36) pour traiter des sorties dudit réseau de capteurs (22) afin de déterminer un profil de distribution de pression (112) du patient ;
l'affichage du profil de distribution de pression (112) du patient sur un écran d'affichage (48) supporté sur le support de patient (36) ;
la communication du profil de distribution de pression (112) du patient hors du lit à un emplacement distant ;
le fait de déterminer, à partir dudit réseau de capteurs (22), si une condition de cisaillement indésirable existe en détectant un mouvement le long dudit réseau de capteurs (22) d'une zone de pression exercée par le patient contre le réseau de capteurs (22), ladite condition indésirable étant détectée uniquement si ladite zone de pression dépasse une taille prédéterminée, uniquement si une grandeur de pression à l'intérieur de ladite zone de pression dépasse un seuil prédéterminé, et uniquement si un taux du mouvement dépasse un seuil prédéterminé ;
l'émission d'une alerte si ladite condition indésirable est détectée.

14. Procédé selon la revendication 13, comportant en outre :
l'utilisation dudit réseau de capteurs (22) et du dispositif de commande (28) pour détecter quand un patient est sorti du support de patient (36) ; et
la communication d'un signal d'alerte du support de patient (36) à l'emplacement distant indiquant que le patient est sorti du support de patient (36).
